# EUROPEAN PATENT SPECIFICATION

(11) **EP 0 941 737 B1**
(45) Date of publication and mention of the grant of the patent: **09.06.2004**
(21) Application number: 97909728.4
(22) Date of filing: 31.10.1997
(51) Int. Cl.: A61K 35/78, A61K 35/80, A61K 35/84, A61K 35/66, A61K 35/56, A23L 1/30, A61P 35/00

(54) **APOPTOSIS INDUCERS**
INDUKTOREN DER APOPTOSE
INDUCTEURS D'APOPTOSE

(30) Priority: 08.11.1996 JP 31122496; 26.12.1996 JP 35641696
(43) Date of publication of application: 15.09.1999
(73) Proprietor: TAKARA BIO INC., Otsu-shi, Shiga (JP)
(72) Inventor: SAKAI, Takeshi-Biomedical Group, Hirosaki-shi,Aomori 036 (JP); KOYAMA, Nobuto-Central Research Laboratories, Otsu-shi,Shiga 520-21 (JP); TATSUMI, Yoko-Central Research Laboratories, Shiga 520-21 (JP); SAGAWA, Hiroaki-Central Research Laboratories, Shiga 520-21 (JP); YU, Fu-Gong-Biomedical Group, Hirosaki-shi,Aomori 036 (JP); IKAI, Katsushige-Central Research Laboratories, Shiga 520-21 (JP); KATO, Ikunoshin-Central Research Laboratories, Shiga 520-21 (JP)
(74) Representative: Wakerley, Helen Rachael
(86) International application number: PCT/JP1997/003997
(87) International publication number: WO 1998/020884

(56) References cited:
- EP-A- 0 693 547
- WO-A-95/20944
- JP-A- 6 025 002
- JP-A- 6 072 888
- JP-A- 7 149 786
- JP-A- 8 169 891
- JP-A- 60 019 716
- PATENT ABSTRACTS OF JAPAN vol. 1995, no. 09, 31 October 1995 (1995-10-31) & JP 07 155151 A (TOKUSHIMA PREF GOV), 20 June 1995 (1995-06-20)
- MURAKAMI AKIRA ET AL: "Glyceroglycolipids from Citrus hystrix, a traditional herb in Thailand, potently inhibit the tumor-promoting activity of 12-O-tetradecanoylphorbol 13-acetate in mouse skin." JOURNAL OF AGRICULTURAL AND FOOD CHEMISTRY, vol. 43, no. 10, 1995, pages 2779-2783, XP002100385 ISSN: 0021-8561

## Description

### Technical Field of the Invention

The present invention relates to pharmaceutically useful apoptosis inducing agents and anticancer agents containing physiologically active substance as effective component(s) derived from animals, microorganisms or plants useful for improving the health; to a functional food or beverage containing said physiologically active substance; and to a method for the manufacture of them.

### Prior Art

In recent years, a mode of apoptosis has been drawing the attention concerning the death of cell tissues.

Unlike necrosis which is a pathological cell death, it is believed that apoptosis is a death which is integrated from the first in gene of the cell itself. Thus, it is believed that gene which programs the apoptosis is activated where some external or internal cause acts as a trigger, a programmed death gene protein is biosynthesized based upon said gene and the cell itself is decomposed by the produced programmed death protein whereby death of the cell is resulted.

If such apoptosis can be expressed in desired tissues or. cells, it will be now possible to exclude the unnecessary or pathogenic cells from living body in their natural form and that will be significantly meaningful.

In recent years, the relation between apoptosis and sphingolipid such as ceramide has been drawing the attention and it has been reported that a *de novo* synthesis of ceramide takes place by palmitic acid and stearic acid whereby apoptosis is induced [*Journal of Biological Chemistry*, volume 272, pages 3324-3329 (1997)].

### Problems to be Solved by the Invention

Although ceramide and free fatty acids are expected to be developed as apoptosis inducing agents, an apoptosis inducing action of other fat-soluble substances derived from living body has been entirely ambiguous.

An object of the present invention is to develop a highly-safe extracted fraction derived from plants, microorganisms or animals having an apoptosis inducing action and to offer an apoptosis inducing agent and an anticancer agent containing said fraction, a functional food or beverage containing said fraction as a constituting component and a method for manufacturing them.

### Means to Solve the Problems

The present inventors have conducted an intensive investigation to achieve such an object and, as a result, they have found that glycerolipid obtained from plants, microorganisms or animals exhibit strong apoptosis inducing action and anticancer action whereupon the present invention has been accomplished.

Thus, the first feature of the present invention relates to the use of glycerolipid consisting of fatty acid and glycerol in residues in the manufacture of a medicament for the treatment of cancer.

The second feature of the present invention relates to the use of glycerolipid consisting of fatty acid and glycerol residues in the manufacture of a food or beverage for the treatment of cancer.

### Brief Explanation of the Drawings

Fig. 1 is a drawing which shows an eluting pattern of the inner solution after dialysis by a DEAE-Sepharose Fast Flow column.
Fig. 2 is a drawing which shows an apoptosis inducing action of the fraction B and the pure substance.
Fig. 3 is a drawing which shows an NMR spectrum of the pure substance.
Fig. 4 is a drawing which shows a total ion chromatogram of the sample A.
Fig. 5 is a drawing which shows a total ion chromatogram of the sample B.
Fig. 6 is a drawing which shows a Sepharose LH-60 column chromatography.
Fig. 7 is a drawing which shows a total ion chromatogram of the lipid component of the fraction No. 20.
Fig. 8 is a drawing which shows a total ion chromatogram of the lipid component of the fraction No. 21.
Fig. 9 is a drawing which shows a total ion chromatogram of the lipid component of the fraction No. 23.
Fig. 10 is a drawing which shows a total ion chromatogram of the sugar component of the fraction No. 20.
Fig. 11 is a drawing which shows a total ion chromatogram of the sugar component of the fraction No. 21.
Fig. 12 is a drawing which shows a total ion chromatogram of the sugar component of the fraction No. 23.
Fig. 13 is a drawing which shows a total ion chromatogram of the lipid component of the fraction eluted with chloroform.
Fig. 14 is a drawing which shows a total ion chromatogram of the lipid component of a fraction eluted with chloroform and acetone in a ratio of 80:20.
Fig. 15 is a drawing which shows a total ion chromatogram of the sugar component of the fraction eluted with chloroform.
Fig. 16 is a drawing which shows a total ion chromatogram of the sugar component of a fraction eluted with chloroform and acetone in a ratio of 80:20.
Fig. 17 is a drawing which shows a total ion chromatogram of the lipid component of a spot where the Rf value is about 0.25.
Fig. 18 is a drawing which shows a total ion chromatogram of the sugar component of a spot where the Rf value is about 0.25.
Fig. 19 is a drawing which shows a total ion chromatogram of the lipid component of a spot where the Rf value is about 0.33.
Fig. 20 is a drawing which shows a total ion chromatogram of the sugar component of a spot where the Rf value is about 0.33.
Fig. 21 is a drawing which shows a total ion chromatogram of the lipid component of an ethanolic extract of *bunashimeji* (*Lyophyllum ulmarium*).
Fig. 22 is a drawing which shows a total ion chromatogram of the sugar component of an ethanolic extract of *bunashimeji*.
Fig. 23 is a drawing which shows a total ion chromatogram of the lipid component of an ethanolic extract of *matcha* (powdered green tea).
Fig. 24 is a drawing which shows a total ion chromatogram of the sugar component of an ethanolic extract of *matcha*.
Fig. 25 is a drawing which shows a total ion chromatogram of the lipid component of a 75% ethanolic extract of rice bran.
Fig. 26 is a drawing which shows a total ion chromatogram of the sugar component of a 75% ethanolic extract of rice bran.

### Mode for Carrying Out the Invention

The present invention will be specifically illustrated as hereinafter.

There is no particular limitation for the glycerolipid used in the present invention provided that they/it are/is free from phosphate ester and phosphonate ester in molecular structure, and they/it may be manufactured from plants, microorganisms or animals or may be synthesised chemically.

There is no particularly limitation for the plants, microorganisms or animals which can be used in the present invention so far as they are glycerolipid-containing things which contain glycerolipid . Examples of the plant are vegetables such as spinach, carrot and onion; dicotyledonous plants such as tea; monocotyledonous plants such as barley and rice; spice materials such as pepper, nutmeg, garlic, bay leaf, celery, mustard, ginger, red pepper, thyme, saffron, cinnamon, vanilla, all spice, *karashi* (Japanese mustard), watercress, *sansho* (Japanese pepper), beefsteak plant, herb, star anise, basil, *nira* (Japanese leek), hops and *wasabi* (Japanese horseradish); products processed from those plants such as cereal residues; algae such as brown algae (*Phaeophycease*), red algae (*Rhodophyceae*), green algae (*Chlorophyceae*) and unicellular green algae; microorganisms such as mushrooms, yeast, filamentous fungi (e.g. *Aspergillus*) and bacteria (e.g. *Bacillus natto* and lactic acid bacteria); and animals such as vertebrates and invertebrates.

The term "tea" used in the present specification may be anything which is usually called tea and its examples are non-fermented tea such as green tea, *sencha* (green tea of middle grade), *bancha* (tea of low grade), *hojicha* (roasted tea) and *matcha* (powdered green tea); semi-fermented tea such as paipao tea, oolong tea and paochong tea; and after-fermented tea such as puer tea. Besides them, the term "tea" covers mate tea, *kuko* (Chinese matrimony vine) tea, adlay tea and barley tea.

There is no particular limitation for the mushrooms used for the present invention although those which are commonly eaten is preferred and the examples are *Basidiomysetes* such as *bunashimeji* (*Lyophyllum ulmarium*), *hatakeshimeji* (*Lyophyllum decastes*)*, shiitake* (*Lentinus edodes*), *matsutake* (*Tricholowa matsutake*), *honshimeji (Lyophyllum shimeji*), *enokitake* (*Flammulina velutipes*), *nameko* (*Pholiota nameko*), *iwatake* (*Gyrophora esculenta*), *kikurage* (*Auricularia auricula*), *kinugasadake* (*Dictyophora indusiata*), *kuritake* (*Naematoloma sublateritium*), *kurokawa* (*Boletopsis leucomelas*), *koutake* (*Sarcodon aspratus*), *shoro* (*Rhizopogon rubescens*), *tamagodake* (*Amanita hemibapha*), *chichitake* (*Lactarius volemus*), *naratake* (*Armillaria mellea*), *hatsutake* (*Lactarius hatsudake*), *hiratake* (*Pleurotus ostreatus*), *maitake* (Grifola frondosa), *matsuoji* (*Lentinus lepideus*) and common mushroom (*Agaricus bisporus* or the like); and *Ascomycetes* such as *tochukaso* (vegetative wasp or plant worm).

There is no particular limitation for the algae used in the present specification and its examples are brown algae such as *fukuronori* (*Colpomenia sinuosa*), *mozuku* (*Nemacystus decipiens* and other species in *Chordariaceae*), *makombu* (*Laminaria japonica*), *gagome kombu* (*Kjellmaniella crassifolia*), *wakame* (*Undaria pinnatifida*), *arame* (*Eisenia pinnatifida*) and *hijiki* (*Hizikia fusiforme*); red algae such as *tengusa* (*Gelidium*) and *igisu* (*Ceramium kondoi*); green algae such *aosa* (*Ulva pertusa*), *kawanori* (*Prasiola japonica*) and *miru* (*Codium fragile*); and unicellular green algae such as *kurorera* (*Chlorella*).

There is no particular limitation for the cereal residue used in the present invention and its examples are rice bran, wheat bran, barley root and *okara* (lees of bean curd).

It is preferred in the present invention that glycerolipid is prepared from those tea, mushrooms, algae or cereal residue and used as effective component(s) for an apoptosis inducing agent, an anticancer agent or a functional food or beverage.

There is no particular limitation for the glycerolipid having an apoptosis inducing action used in the present invention and examples of the applicable glycerolipid are mono-, di- and tri-acylglycerol

Examples of the fatty acid which constitutes the glycerolipid used in the present invention are saturated and/or unsaturated fatty acids such as tetradecanoic acid (myristic acid, C14:0), hexadecanoic acid (palmitic acid, C16:0), tetradecenoic acid (myristoleic acid, C14:1), hexadecenoic acid (palmtoleic acid, C16:1), octadecenoic acid (oleic acid, C18:1), cis-9,cis-12-octadecadienoic acid (linoleic acid, C18:2) and 9,12,15-octadecatrienoic acid (linolenic acid, C18:3).

The glycerolipid having an apoptosis inducing action used in the present invention derived from plants, microorganisms or animals can be easily manufactured by a method in which a step of extracting with an organic solvent such as water-containing hydrophilic organic solvent (for example, aqueous ethanol) is included. However, there is no particular limitation for the manufacturing method for said glycerolipid . Thus, not only a mere extraction with water but also known methods which can be used for purification of said glycerolipid may be used. It is also possible that a substance which contains glycerolipid derived from plants, microorganisms or animals is treated chemically, physically or enzymatically and then the aimed glycerolipid is purified. Upon purification, the physicochemical property, or physiological activity (apoptosis inducing action, etc.) of glycerolipid may be used as an index for the purification.

In the present invention, glycerolipid derived from plants, microorganisms or animals are/is extracted with an organic solvent such as hexane, chloroform, ethyl acetate, a hydrophilic organic solvent (for example, acetone, propanol, ethanol and methanol) and a mixture thereof or a mixed solvent thereof with water and an'edible substance containing glycerolipid can be easily manufactured by a manufacturing method including a step of extracting, for example, with an aqueous ethanol. When used as food or beverage, extraction is preferably conducted under such an extracting condition where an organic solvent, particularly a hydrophilic organic solvent such as 10-95% or, preferably, 20-80% aqueous ethanol is used usually for several minutes to several days or, preferably, for several tens minutes to several tens hours usually at 10∼70°C or preferably at 20~60 °C whereupon glycerolipid which are/is apoptosis inducing compound(s) can be efficiently extracted. When glycerolipid derived from plants, microorganisms or animals are/is treated with an acid or an alkali prior to the extraction with organic solvent or, particularly, with hydrophilic organic solvent, it is possible that glycerolipid which are/is apoptosis inducing compound(s) can be extracted more efficiently.

With regard to methods for collecting pure glycerolipid which are/is used in the present invention, there are known methods such as a Bligh-Dyer method and Folch partition method (cf. " *Shin Seikagaku Jikken Koza* (New Textbook on Experiments of Biochemistry)", Volume 4, Lipid III, pages 104-109, 1990, edited by Japan Biochemical Society, published by Tokyo Kagaku Dojin) and any of them suitable for the object can be used for extraction of the glycerolipid used in the present invention.

In the purification of the glycerolipid used in the present invention, the above-mentioned extract is subjected to a chromatography using a hydrophobic carrier, a reversed phase carrier, a normal phase carrier, etc. whereby more efficient separation can be carried out.

There is no particular limitation for the hydrophobic carrier but any of the known carriers may be used. Examples of such a carrier are the resin of a Sephacryl type, a Sepharose type, a Toyopearl type, etc. wherein butyl, octyl or phenyl group is introduced and an Amberlite adsorption resin XAD-1, - 2, -4, -200, -7 or -8. There is no particular limitation for the reversed phase carrier but any of the known carriers may be used and an example of such a carrier is silica gel in which an alkyl chain is covalently bonded. There is no particular limitation for the normal phase carrier but any of the known carriers may be used and an example of said carrier is silica gel. In conducting the purification, physical and chemical properties of the glycerolipid or physiological activity such as an apoptosis inducing action may be used as an index.

The glycerolipid derived from plants, microorganisms or animals used in the present invention may, for example, be purified from an aqueous ethanolic extract of edible plants, edible microorganisms, edible animals, etc. by means of conventional ion exchange resin treatment, hydrophobic chromatographic treatment, gel filtration treatment, etc.

Usually, there are many cases where the glycerolipid in plants, microorganisms or animals are/is present in a form of a high-molecular substance bonded with saccharide and/or protein near membrane and, upon extraction with hot water for example, a substance containing glycerolipid in which the glycerolipid are/is bonded with saccharide and/or protein can be obtained. Incidentally, the term saccharide means a saccharide which is present in plants, microorganisms or animals and it covers monosaccharide, oligosaccharide, polysaccharide and glycoconjugates.

In the present invention, the high-molecular substance containing the glycerolipid prepared as such may be used as it is although, depending upon the object for use, saccharide and/or protein are/is treated enzymatically, chemically and/or physically to purify and collect the glycerolipid whereby it is possible to obtain the glycerolipid in a more purified state.

The glycerolipid which are/is obtained by the above-mentioned method and used in the present invention are/is the highly safe substance(s) obtained from nature and particularly useful as food and/or beverage.

When the resulting glycerolipid are/is bonded to ligand which recognizes the target cells such as monoclonal antibody, it is possible to induce the apoptosis in any target cells specifically. In addition, when the target cells are cancer cells, it is possible to achieve an anticancer action as a result of induction of apoptosis in the desired cancer cells. Further, when the compound of the present invention is bonded to a carrier such as albumin, a substance having a higher absorbing property can be offered.

The apoptosis inducing agent of the present invention may be made into a pharmaceutical preparation by combining the glycerolipid such as those/that derived from plants, microorganisms or animals which are/is effective component(s) with a known pharmaceutical carrier. Generally, the apoptosis inducing compound(s) of the present invention such as glycerolipid are/is compounded with a pharmaceutically acceptable liquid or solid carrier followed, if necessary, by adding solvent, dispersing agent, emulsifier, buffer, stabilizer, vehicle, binder, disintegrating agent, lubricant, etc. thereto whereupon a solid preparation such as tablets, granules, diluted powder, pulverized preparation and capsules and liquid preparation such as normal liquid preparation, suspension and emulsion can be prepared. Alternatively, the compound(s) may be made into a dry preparation which can be converted into liquid by addition of a suitable carrier thereto before actual use.

The apoptosis inducing agent of the present invention may be administered by means of any of oral agents and parenteral agents such as injection and instillation agents.

Pharmaceutical carriers may be selected depending upon the above-mentioned dosage forms and preparation forms. In the case of oral agents, starch, lactose, sucrose, mannitol, carboxymethylcellulose, corn starch, inorganic salts, etc. may be used. In preparing the oral agents, binder, disintegrating agent, surfactant, lubricant, fluidity promoter, corrigent, coloring agent, perfume, etc. may be further compounded therewith.

On the other hand in the case of parenteral agents, the glycerolipid derived, for example, plants, microorganisms or animals which are/is effective component(s) in the present invention having an apoptosis inducing action are/is dissolved or suspended in a diluent such as distilled water for injection, physiological saline solution, aqueous solution of glucose, vegetable oil for injection, sesame oil, peanut oil, soybean oil, corn oil, propylene glycol and polyethylene glycol followed, if necessary, by adding bactericide, stabilizer, isotonic agent, anesthetizing agent, etc. thereto.

The apoptosis inducing agent of the present invention may be administered by an appropriate administering route depending upon the preparation forms. There is no particular limitation for the method of administration as well and any of internal and external administrations and injection may be applied. Injection preparations may, for example, be administered intravenously, intramuscularly, subcutaneously, intracutaneously, etc. External preparation covers suppository and the like.

Dose of the apoptosis inducing agent of the present invention may be appropriately established according to preparation form, method of administration and object of administration as well as age, body weight and symptom of the patient to whom the inducing agent is administered and is not fixed although, usually, the amount of the effective component contained in the preparation is 0.1∼200 mg/kg per day for adult. It goes without saying that the dose varies depending upon various conditions and, therefore, in some cases, less dose than the above range may be sufficient while, in other cases, more dose than the above range may be necessary. The pharmaceutical agent of the present invention is not only orally administered as it is but also may be taken daily by adding it to any food or beverage.

The glycerolipid used in the present invention have/has an activity of suppressing the growth of cancer cells. The action mechanism of the glycerolipid used in the present invention for suppressing the growth of cancer cells does not limit the present invention at all and, for example, an apoptosis inducing action to cancer cells is covered by the present invention as well.

The glycerolipid having an anticancer action such as glycerolipid derived, for example, from plants, microorganisms or animals are/is used as effective component (s) and made into a pharmaceutical preparation by compounding with a known pharmaceutical carrier whereupon an anticancer agent can be prepared. Manufacture of the anticancer agent may be conducted in accordance with the above-mentioned method. Generally, the glycerolipid are/is compounded with a pharmaceutically acceptable liquid or solid carrier followed, if necessary, by adding solvent, dispersing agent, emulsifier, buffer, stabilizer, vehicle, binder, disintegrating agent, lubricant, etc. thereto whereupon a solid preparation such as tablets, granules, diluted powder, pulverized preparation and capsules and a liquid preparation such as normal liquid preparation, suspension and emulsion can be prepared. Alternatively, the compound may be made into a dry preparation which can be converted into liquid by addition of a suitable carrier before actual use.

The anticancer agent may be administered by means of any of oral agents and parenteral agents such as injection and instillation agents.

Pharmaceutical carriers may be selected depending upon the above-mentioned dosage forms and preparation forms and may be used in accordance with the above-mentioned case of the apoptosis inducing agent.

The anticancer agent of the present invention may be administered by an appropriate administering route corresponding to the preparation forms. There is no particular limitation for the method of administration as well and any of internal and external administrations and injection may be applied. Injection preparations may, for example, be administered intravenously, intramuscularly, subcutaneously, intracutaneously, etc. External preparation covers suppository and the like.

Dose of the anticancer agent of the present invention may be appropriately established according to preparation form, method of administration and object of administration as well as age, body weight and symptom of the patient to whom the inducing agent is administered and is not fixed although, usually, the amount of the effective component contained in the preparation is 0.1∼200 mg/kg per day for adult. It goes without saying that the dose varies depending upon various conditions and, therefore, in some cases, less dose than the above range may be sufficient while, in other cases, more dose than the above range may be necessary. The pharmaceutical agent of the present invention is not only orally administered as it is but also may be taken daily by adding it to any food or beverage.

The pharmaceutical agent of the present invention can be used as a therapeutic agent for cancer diseases and the like. In addition, the method for the induction of apoptosis offered by the apoptosis inducing agent of the present invention is useful in investigating the mechanism of biophylaxis, function of immune or relation with diseases such as cancer and also in developing the inhibitor of induction of apoptosis. Particularly, the glycerolipid which are/is apoptosis inducing compound(s) prepared from plants, microorganisms or animals having long history as food have/has high safety when administered orally. In addition, food or beverage in which the glycerolipid of the present invention such as those/that derived from plants, microorganisms or animals are/is contained therein, added thereto and/or diluted therein has naturally high safety and it is quite useful for improvement in symptoms and prevention of gastrointestinal cancer or the like because of its apoptosis inducing action and anticancer action due to the apoptosis inducing action.

There is no particular limitation for the food or beverage of the present invention and its examples are processed agricultural/forestry products, processed livestock products, processed fishery product, etc. including processed cereal products such as processed wheat products, processed starch products, processed premix products, noodles, macaroni, bread, bean pastes, *soba* (Japanese buckwheat noodles), *fu* (Japanese wheat-gluten bread), *bifun* (Chinese bean jelly sticks), *harusame* (Japanese bean jelly sticks) and packed *mochi* (rice cake); processed fat/oil products such as plastic fat/oil, oil for *tempura* (deep-fried food), salad oil, mayonnaise and dressing; processed soybean products such as *tofu* (soybean curd), *miso* (fermented soybean paste) and *natto* (fermented soybeans); processed meat products such as ham, bacon, press ham and sausage; fishery products such as frozen *surimi* (ground fish meat), *kamaboko* (boiled fish paste), *chikuwa* (Japanese fish paste cooked in a bamboo-like shape), *hampen* (light fish cake), *satsumaage* (fried fish balls), *tsumire* (boiled fish paste with some ingredients), *suji* (fish products), fish meat ham, sausage, *katsuobushi* (dried bonito), processed fish egg products, canned fishery products and *tsukudani* (preserved fish food boiled down in soy sauce) ; milk products such as milk, cream, yogurt, butter, cheese, condensed milk, powdery dry milk and ice cream; processed vegetable/fruit products such as pastes, jams, pickles, fruit beverages, vegetable beverages and mixed beverages; confectionery such as chocolate, biscuit, bun, cake, *mochigashi* (rice ball cake) and rice cracker; alcoholic beverages such as *sake* (Japanese rice wine), Chinese wine, wine, whisky, *shochu* (Japanese distilled liquor), vodka, brandy, gin, ram, beer, soft alcoholic beverages, fruit wine and liquor; table luxuries such as green tea, black tea, oolong tea, coffee, soft drinks and lactic acid beverages; seasonings such as soybean sauce, Worcester sauce, vinegar and *mirin* (sweet *sake*); spices such as extracts of garlic, pepper, red pepper, nutmeg, ginger, star anise, herb and basil), canned, bottled or bagged food such as boiled rice assorted with seasoned beef, *kamameshi* (boiled rice packed in a small kettle-shaped container), *sekihan* (rice boiled with red beans), curry and rice, and other already-processed food; semi-dried or concentrated food such as liver paste and other spread, soup for *soba* and *udon* (both being Japanese noodles) and concentrated soup; dried food such as instant noodles, instant curry, instant coffee, powdery juice, powdery soup, instant *miso* soup, retort food, retort beverage and retort soup; frozen food such as *sukiyaki, chawan-mushi* (pot-steamed hotchpotch), *unagi kabayaki* (broiled eels), hamburg steak, *shao-mai* (Chinese steamed dumpling), *gyoza* (fried dumpling stuffed with minced pork, etc.), various kinds of sticks and fruit cocktail; solid food; liquid food such as soup; and spices.

There is no particular limitation for the manufacturing method of food or beverage of the present invention and its examples are cooking, processing and commonly-used method for the manufacture of food or beverage where any method may be used so far as the food or beverage manufactured as such contains the glycerolipid such as those/that derived from plants, microorganisms and animals which are/is apoptosis inducing compound(s).

With regard to cooking and processing, it will do that the product after said cooking or processing contains the glycerolipid having an apoptosis inducing property.

Thus, the glycerolipid having an apoptosis inducing property may be added before, during or after the cooking or processing. Alternatively, cooked or processed product or a raw material therefor may be added to a substance containing the glycerolipid so that said glycerolipid are/is diluted.

In the manufacture of the food or beverage, the glycerolipid having an apoptosis inducing action may be added in any of the steps. With regard to a method of addition, said glycerolipid may be added or food, beverage or a material therefor may be added to said glycerolipid so that said glycerolipid are/is diluted. The addition may be conducted either at a time or for several times. Accordingly, the food or beverage having an apoptosis inducing action can be easily manufactured. When any of those steps is applied, the food or beverage containing the glycerolipid having an apoptosis inducing property or the food or beverage where the glycerolipid of the present invention are/is added thereto or diluted therein is defined as the food or beverage of the present invention.

There is no particular limitation for the amount of the glycerolipid of the present invention having an apoptosis inducing action (such as the glycerolipid which are/is the apoptosis inducing compound(s) of the present invention derived from plants, microorganisms or animals) in the food but the amount may be appropriately selected from the viewpoint of organolepticity and physiological activity. For example, the amount of said glycerolipid is not less than 10⁻⁹ part per 100 parts of the food and, in view of organolepticity and physiological action as food, it is preferably from 10⁻⁸ part to 5 parts and, more preferably, from 10⁻⁷ part to 2 parts.

There is no particular limitation for the amount of the glycerolipid of the present invention having an apoptosis inducing action (such as the glycerolipid which are/is the apoptosis inducing compound(s) of the present invention derived from plants, microorganisms or animals) in the beverage but the amount may be appropriately selected from the viewpoint of organolepticity and physiological activity. For example, the amount of said glycerolipid is not less than 10⁻⁹ part per 100 parts of the beverage and, in view of organolepticity and physiological action as beverage, it is preferably from 10⁻⁸ part to 5 parts and, more preferably, from 10⁻⁷ part to 2 parts. Incidentally, in the present specification, the term "part (s)" stands for that/those by weight.

There is no particular limitation for the shape of the food or beverage of the present invention so far as the glycerolipid of the present invention having an apoptosis inducing property are/is contained therein, added thereto and/or diluted therein but may cover any shape which can be taken orally such as tablets, granules, capsules, gel and sol.

The food or beverage of the present invention contains a large amount of the glycerolipid which are/is apoptosis inducing compound(s) of the present invention having a physiological activity and is, particularly, a healthy food or beverage useful for maintaining good health of stomach and intestine due to the apoptosis inducing action of said glycerolipid.

The anticancer food or anticancer beverage where the glycerolipid of the present invention such as those/that derived from plants, microorganisms or animals are/is contained therein, added thereto and/or diluted therein may be manufactured according to the above-mentioned method for the manufacture of an apoptosis inducing food or an apoptosis inducing beverage using the anticancer activity as an index.

The glycerolipid which are/is apoptosis inducing compound (s) of the present invention are/is derived from plants, microorganisms or animals and the use of the glycerolipid particularly derived from edible plants, microorganisms or animals to food or beverage is quite safe where said apoptosis inducing compound (s) show (s) no toxicity to animals at the concentration for achieving the physiological function.

The glycerolipid of the present invention in edible plants, microorganisms or animals have/has a long history as a food and the substance containing said glycerolipid according to the present invention prepared therefrom have/has a quite high safety when administered orally. Accordingly, it is natural that the food or beverage where said glycerolipid are/is added thereto and/or diluted therein has a high safety.

It has now been clarified that, as mentioned above, the glycerolipid which are/is lipid(s) available in living body according to the present invention have/has an apoptosis inducing activity.

The glycerolipid which are/is apoptosis inducing compound(s) used in the present invention are/is present in large quantities in membrane fractions of plants, microorganisms or animals and, particularly, they/it can be manufactured in a low cost and in an easy manner from edible plants or microorganisms. In addition, the glycerolipid which are/is the aimed apoptosis inducing compound(s) can be selectively purified by selecting an appropriate solvent according to the degree of hydrophobicity thereof.

The glycerolipid which are/is the apoptosis inducing compound(s) used in the present invention (such as those/that derived from plants, microorganisms or animals) can easily give their/its apoptosis inducing action to food or beverage and the glycerolipid of the present invention having the apoptosis inducing property and anticancer property are/is quite useful as additive (s) to food or beverage.

### Examples

The present invention will now be more specifically illustrated by way of the following examples although the present invention is not limited by those examples at all. Incidentally, the term % in the examples means that by weight.

### Example 1

### Preparation of the Composition Having an Apoptosis Inducing Action from Seaweed

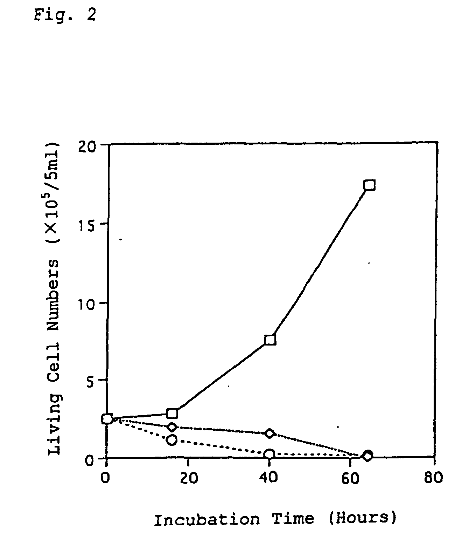

(1) *Gagome kombu* (*Kjellmaniella crassifolia*) was well dried and 20 kg of the dried product was ground by a free grinder (manufactured by Nara Kikai Seisakusho).
   Calcium chloride dihydrate (manufactured by Nippon Soda) (7.3 kg) was dissolved in 900 ml of tap water and then 20 kg of ground *gagome kombu* were mixed therewith. Temperature of the liquid was raised from 12°C to 90°C by bubbling the steam thereinto for 40 minutes and the mixture was kept at 90-95°C for one hour with stirring and cooled to give 1,100 liters of cooled product.
   Then the cooled solution was subjected to a solid-liquid separation using a solid-liquid separator (type CNA; manufactured by Westfalier Separator) to prepare about 900 liters of supernatant liquid after the solid-liquid separation.
   The supernatant liquid (360 liters) after the solid-liquid separation was concentrated to 20 liters using an FE10-FC-FUS 0382 (fractionating molecular weight: 30,000) (manufactured by Daicel). Then 20 liters of tap water were added thereto, the mixture was concentrated to 20 liters and such treatments were repeated for five times to desalt whereupon 25 liters of extract derivative from *gagome kombu*.
   One liter of said solution was freeze-dried to give 13 g of a dried product.
(2) The extract (1.4 liters) mentioned in Example 1-(1) was dialyzed against 20 mM acetate buffer (pH 6) containing 0. 2M CaCl₂ to give an internal liquid after the dialysis. DEAE-Sepharose Fast Flow column (14 cm diameter × 45.5 cm height) was equilibrated with the same buffer, applied with the inner liquid after the dialysis, washed with the same buffer and eluted with said buffer containing 2M NaCl by means of a concentration gradient method. Total sugar amount and uronic acid amount were determined by means of a phenol-sulfuric acid method and a carbazole-sulfuric acid method to give fractions A, B and C in the order of elution. They were dialyzed against distilled water and freeze-dried to give 760 mg of a fraction B.
   The fraction B showed an apoptosis inducing activity.

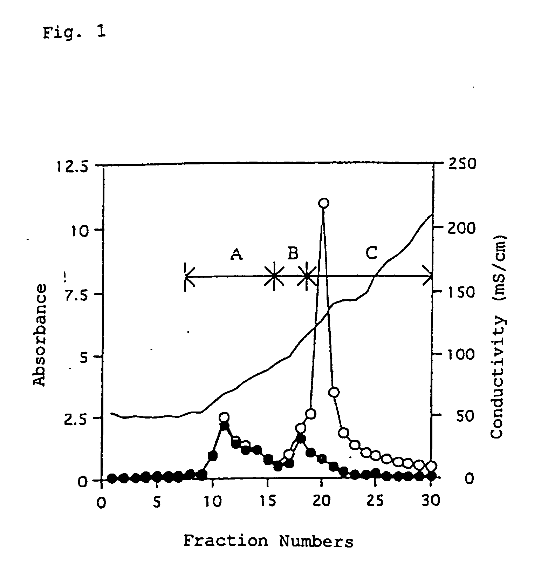

   Fig. 1 shows eluting patters of the extract from the DEAE-Sepharose Fast Flow column. Thus, Fig. 1 is a drawing which shows the eluting patterns of the extract derived from sea algae from the DEAE-Sepharose Fast Flow column (14 cm diameter × 45.5 cm height) in which an ordinate shows absorbance at 530 nm by a carbazole-sulfuric acid method and that at 480 nm by a phenol-sulfuric acid method as well as conductivity (mS/cm) while an abscissa shows fraction numbers. In the drawing, open circles are absorbances at 480 nm by a phenol-sulfuric acid method, black dots are those at 530 nm by a carbazole-sulfuric acid method and a solid line is conductivity. Incidentally, amount of the liquid for one fraction was 1,000 ml.
(3) The fraction B (8.6 g) fractionated by the above method was applied to a Phenyl-Sepharose 6 Fast Flow column (4.4 cm diameter × 20 cm height) which was equilibrated with 1M NaCl wherein the final concentration of NaCl was adjusted to 1M. The column was washed with 1M NaCl and a fraction which was eluted by means of a concentration gradient to water was obtained. This was concentrated by means of a rotary evaporator and dialyzed against distilled water to give about 6.5 ml of pure fraction. The pure fraction was freeze-dried to give 60 mg of pure product. This pure product showed an apoptosis inducing activity.
(4) Measurement of Apoptosis Inducing Action of the Pure Product
   Apoptosis inducing activity of the above fraction B and the pure product mentioned in Example 1-(3) was measured as follows. Thus, 0.5 ml of aqueous solution of the fraction B or of said pure product was added to 2.5 × 10⁵ cells/4.5 ml of HL-60 (ATCC CL-240) cultured in an RPMI 1640 medium containing 10% of fetal calf serum and incubated at 37°C for 48 hours. At the same time, distilled water and an Actinomycin D solution (10 µg/ml) which was known to have an apoptosis inducing activity were added to prepare control samples. Formation of apoptic bodies, contraction of cells and aggregation of nuclei were observed by naked eye under a microscope whereby the living cell numbers were counted. When such phenomena were observed and a reduction in living cell numbers was recognized, it was decided that an apoptosis inducing activity was positive.
   The apoptosis inducing activity was confirmed in the case where the fraction B, the purified product or Actinomycin D was added. The result is given in Fig. 2. Thus, Fig. 2 is a drawing which shows the relation between the incubation time and the living cell numbers in the medium when the fraction B or the pure product was added to the above-mentioned medium of HL-60 cells to an extent of the final concentration of 2 mg/ml or 0.9 mg/ml, respectively. The abscissa is an incubation time (hours) while the ordinate is living cell numbers (× 10⁵/5 ml) in the medium. In the drawing, open squares are the control where water was added, open rhombs are the case where the fraction B was added and open circles are the case where the pure product was added.
(5) Physical and Chemical Properties of the Pure Product Having an Apoptosis Inducing Action
   Physical and chemical properties of the pure product mentioned in the above Example 1-(3) were measured.

### (i) NMR Analysis

¹H-NMR Spectrum was measured using a JNM-A500 nucleomagnetic resonance apparatus (manufactured by Nippon Denshi).

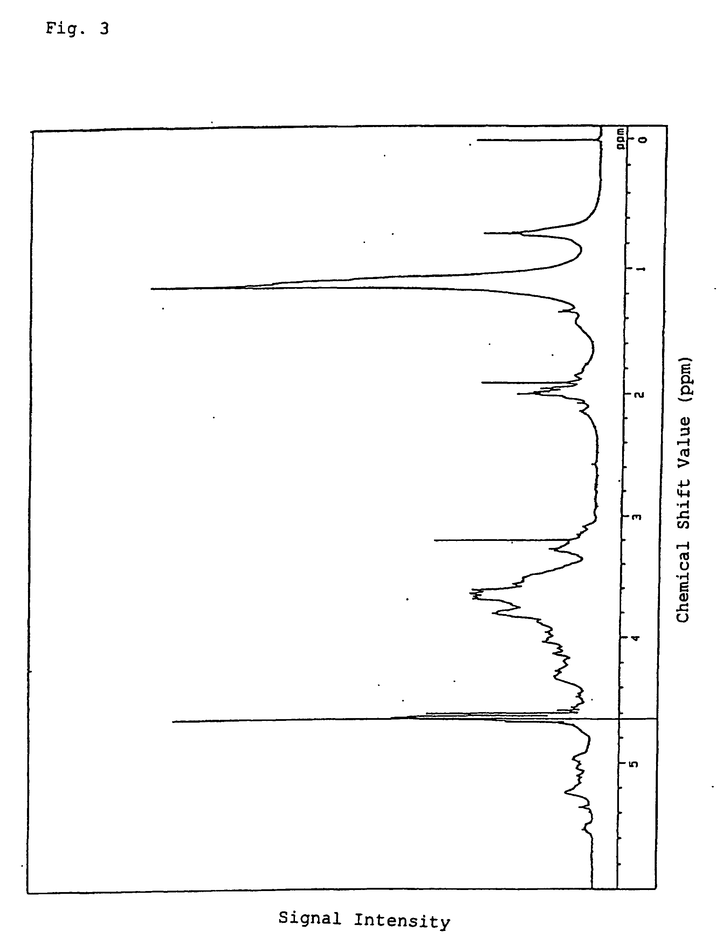

The result is shown by the ¹H-NMR spectrum expressed by Fig. 3. In Fig. 3, the ordinate shows signal intensity while the abscissa shows chemical shift value (ppm). Incidentally, the chemical shift value in the ¹H-NMR was expressed in such a manner that the chemical shift value of HOD was defined as 4.65 ppm.
¹H-NMR (D₂O)
δ 0.7 (H of methyl at the end of the fatty acid), 1.1 (methylene of the fatty acid and H of methyl at C-5 position of fucose), 3.1-5.6 (H derived from the sugar)

### (ii) GC-MS Analysis

A 5% methanolic solution of HCl (1 ml) was added to 2 mg of the pure product mentioned in Example 1-(3) and the mixture was sealed in a tube and heated at 85°C for three hours. After cooled, this was extracted with 1 ml of n-hexane for three times and the resulting extract with n-hexane was concentrated to about 500 µl in a nitrogen stream to give a sample A.

The residual methanolic layer was neutralized by adding silver carbonate thereto and filtered, nitrogen stream was blown onto the filtrate to evaporate to dryness and the residue was dried for four hours using a vacuum pump. After dried, five drops of a TMS reagent (which was prepared as follows; thus, 2.6 ml of hexamethyl disilazane was added to 2.0 ml of dry pyridine, then 1.6 ml of trimethyl chlorosilane were added thereto, the resulting turbid substance was removed by centrifugation and the supernatant liquid obtained thereby was used as the reagent) were added to the residue and the mixture was allowed to stand at room temperature for 30 minutes. After that, the mixture was warmed at 50°C for ten minutes, cooled and stirred after addition of 1 ml of chloroform and the chloroform layer was washed with 1 ml of water for three times. The resulting chloroform solution was used as the sample B.

The above-prepared samples A and B were subjected to a GC-MS analysis under the following conditions using a DX-302 mass spectrometer (manufactured by Nippon Denshi).

### [Sample A]

Column: TC-1 (G. L. Science), 30 m × 0.25 mm inner diameter
Temperature: raised from 130°C to 250°C at a rate of 4°C per minute and kept at 250°C for five minutes
Flow rate: 1.2 ml of helium gas per minute
Mass range for mass spectrum measurement: m/z 50-500
Repeating time for mass spectrum measurement: 3 seconds

### [Sample B]

Column: TC-1 (G. L. Science), 30 m × 0.25 mm inner diameter
Temperature: raised from 130°C to 300°C at a rate of 4°C per minute and kept at 300°C for five minutes
Flow rate: 1.2 ml of helium gas per minute
Mass range for mass spectrum measurement: m/z 50-800
Repeating time for mass spectrum measurement: 3 seconds

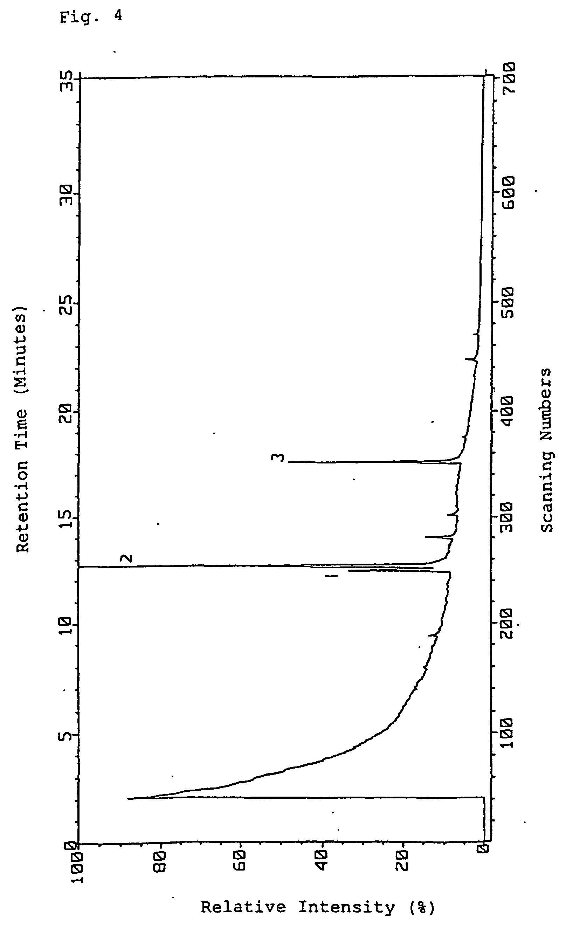

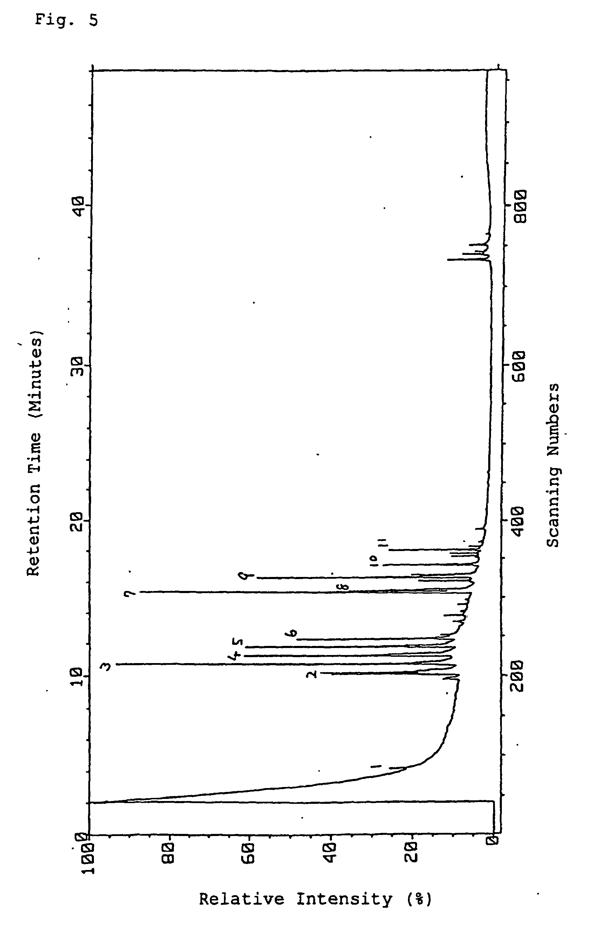

The result was that the samples A and B showed the total ion chromatograms as shown in Fig. 4 and Fig. 5, respectively. In each of the drawings, the ordinate is a relative intensity (%) while the upper and lower parts of the abscissa are retention time (minutes) and scanning numbers, respectively.

Confirmation of the peaks in the chromatograms obtained from the samples A and B was conducted by analysis of the mass spectrum of each of the peaks.

Methyl esters of each of tetradecanoic acid (myristic acid) (peak 2), hexadecanoic acid (palmitic acid) (peak 3) and tetradecenoic acid (peak 1) were confirmed as main components from the sample A.

From the sample B, a peak (peak 1) derived from glycerol, peaks (peaks 2, 3 and 4) derived from fucose, peaks (peaks 5 and 6) derived from xylose, a peak (peak 7) derived from mannose, peaks (peaks 8, 9 and 10) derived from galactose and a peak (peak 11) derived from glucuronic acid were confirmed.

Accordingly, it was found that the above-mentioned pure product contained glycerolipid and/or glyceroglycolipid.

### Example 2

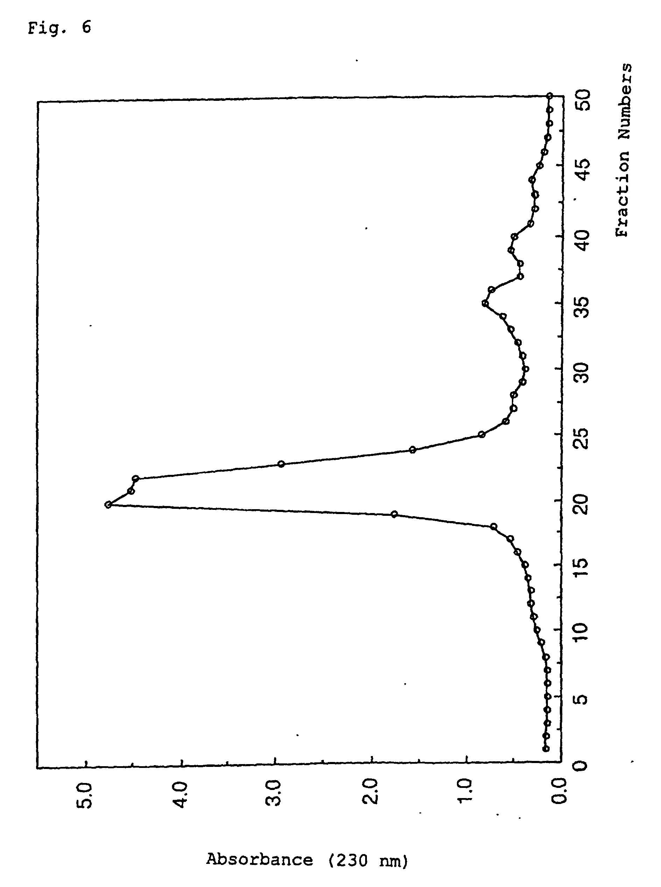

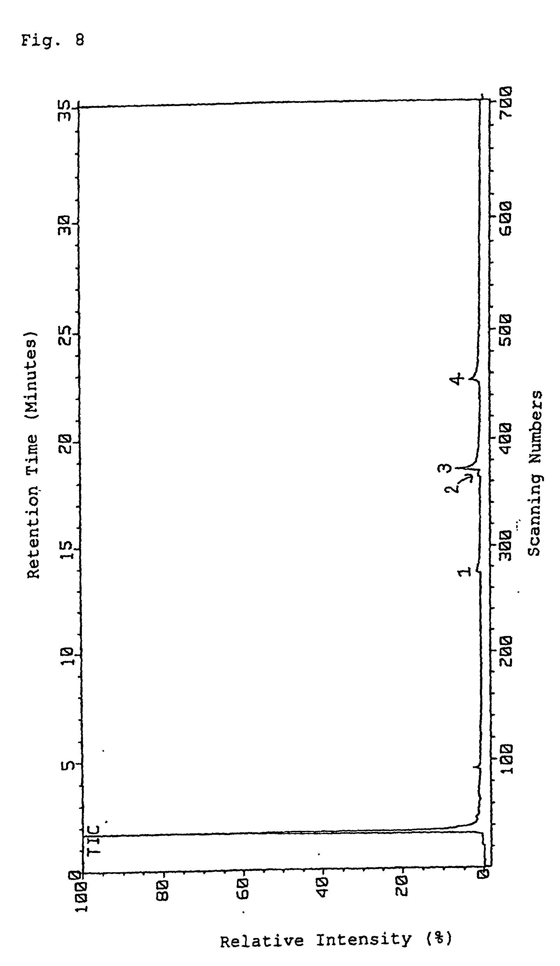

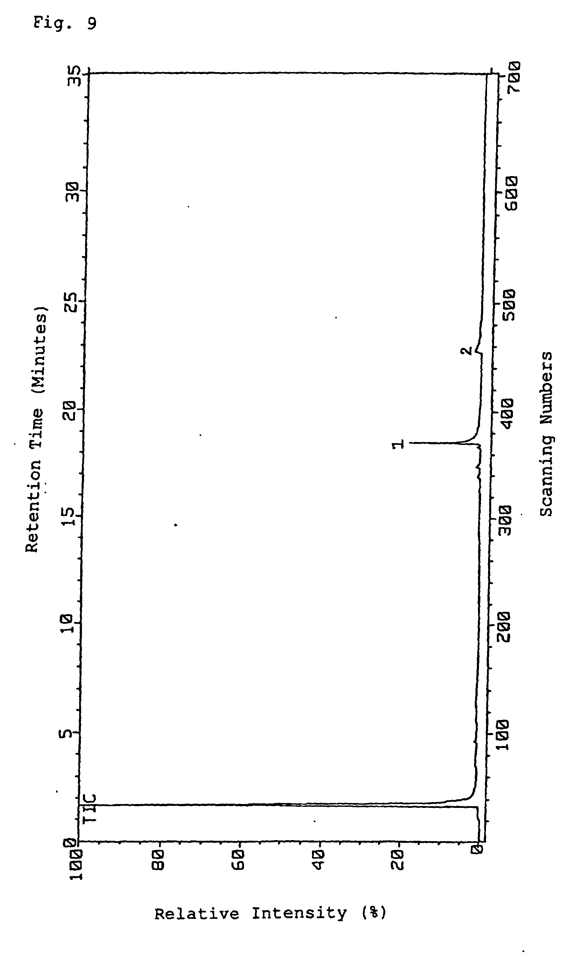

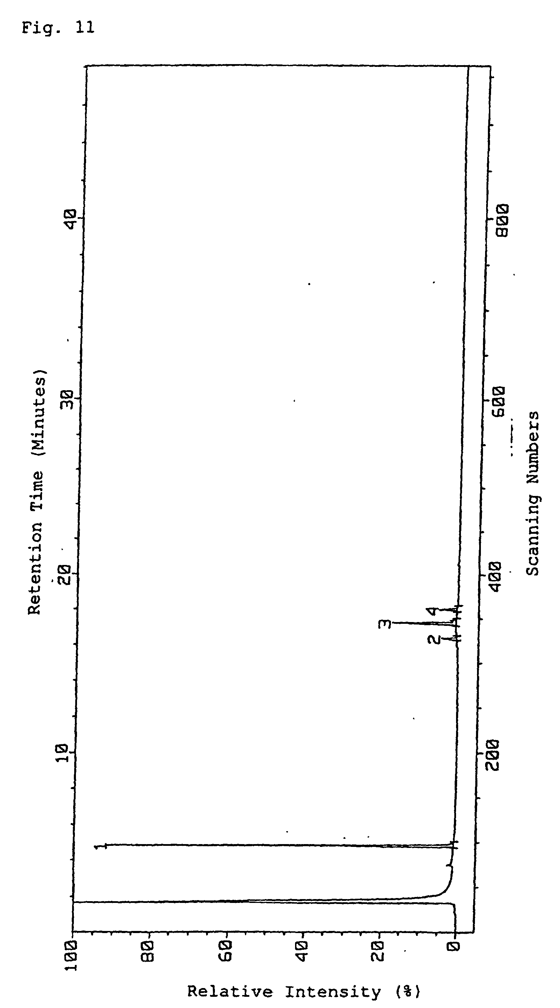

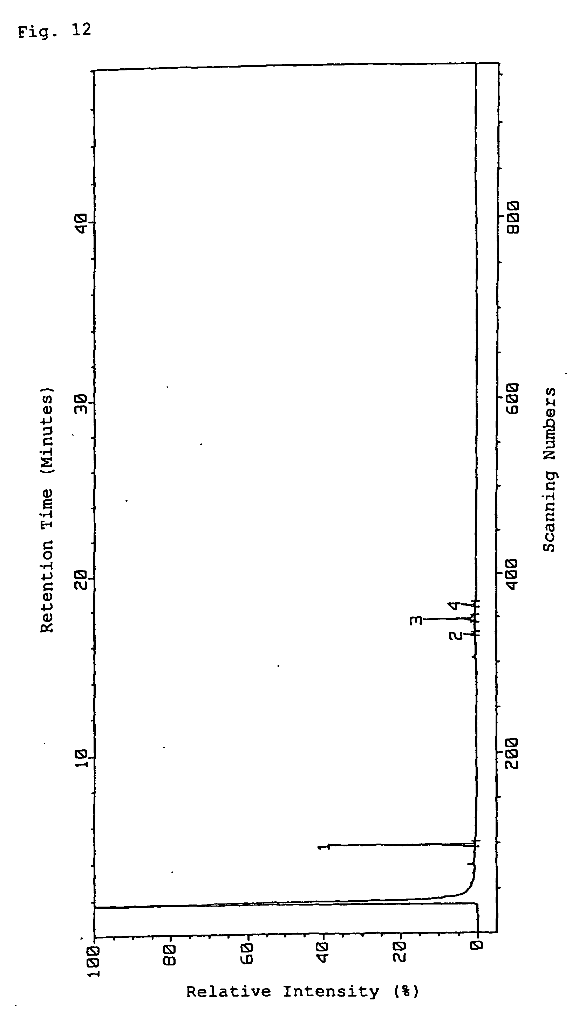

(1) Ground product (2 kg) of dried *gagome kombu* was suspended in 20 liters of 80% ethanol, stirred at 25°C for three hours and filtered to remove the soluble matters. The resulting residue was suspended in 20 liters of a buffer (pH 8.2) which contained 1.5 units of an endo-sulfated-fucose-containing polysaccharide (refer to WO 97/26896), 10% of ethanol, 100 mM of sodium chloride and 50 mM of calcium chloride and stirred at 25°C for 24 hours. Then this stirred solution was filtered and the residue was washed with 10% ethanol containing 50 mM sodium chloride. The washed residue was suspended in 40 liters of a buffer (pH 6.6) containing 4 g of alginic acid lyase (manufactured by Nagase Seikagaku Kogyo), 100 mM of sodium dihydrogen phosphate, 100 mM of sodium chloride and 10% of ethanol and stirred at 25°C for 96 hours. The solution was centrifuged and the resulting supernatant liquid was concentrated using an ultrafilter equipped with a hollow fiber having an exclusion molecular weight of 100, 000 and substituted with 10% ethanol containing 100 mM of sodium chloride. To this solution was added a 0.4 M calcium acetate solution of the same volume, the mixture was stirred for 30 minutes and centrifuged and the resulting supernatant liquid was subjected to an ultrafiltration under the same conditions as above and substituted with 100 mM of sodium chloride. To this solution was added sodium chloride to make its concentration 1M and the pH was adjusted to 8 whereupon 1.4 liters of an extract from *gagome kombu* were obtained.
   The extract was treated with a column of 2.9 liters of Phenylcellulofine (manufactured by Seikagaku Kogyo) and eluted with 6 liters of 1M sodium chloride, 6 liters of water and 8 liter of ethanol in this order.
   The apoptosis inducing action of each of the eluted fractions was measured by a method mentioned in Example 1-(4) whereupon an apoptosis inducing action and a suppressive action to cancer cells were noted in the fraction which was eluted with ethanol.
   The fraction eluted with ethanol was concentrated and evaporated to dryness, the residue was dissolved in ethanol to give a 175 ml solution, 58 ml of the resulting solution were treated with a column of 1,130 ml of Sepharose LH-60 (manufactured by Pharmacia) previously equilibrated with ethanol, the column was eluted with ethanol and the eluate was collected every 60 ml.
   The eluting pattern is given in Fig. 6. Thus, Fig. 6 is a drawing which shows the result of a chromatography with a column of Sepharose LH-60 in which the ordinate is absorbance at 230 nm while the abscissa is fraction numbers.
   The apoptosis inducing activity of the eluted fractions was measured by the method mentioned in Example 1-(4) whereupon it was found that the fractions of fraction Nos. 8-15 and 27-49 had no activity while the fractions of fraction Nos. 16-21 and 22-26 showed an apoptosis inducing action and an action of suppressing the growth of cancer cells.
   Among those fractions showing the potent activity, the three fractions (fraction Nos. 20, 21 and 23) were subjected to analysis of the components by a method mentioned in Example 1.

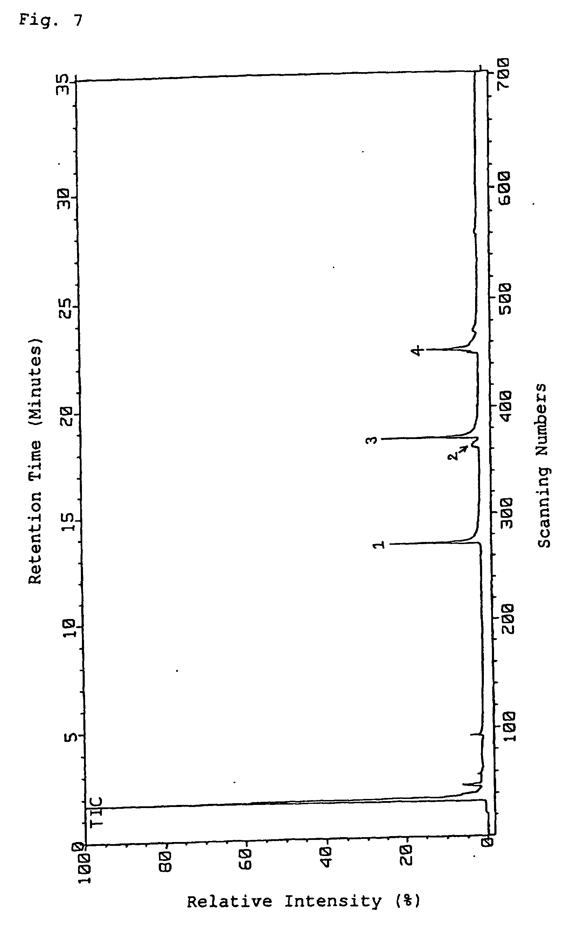

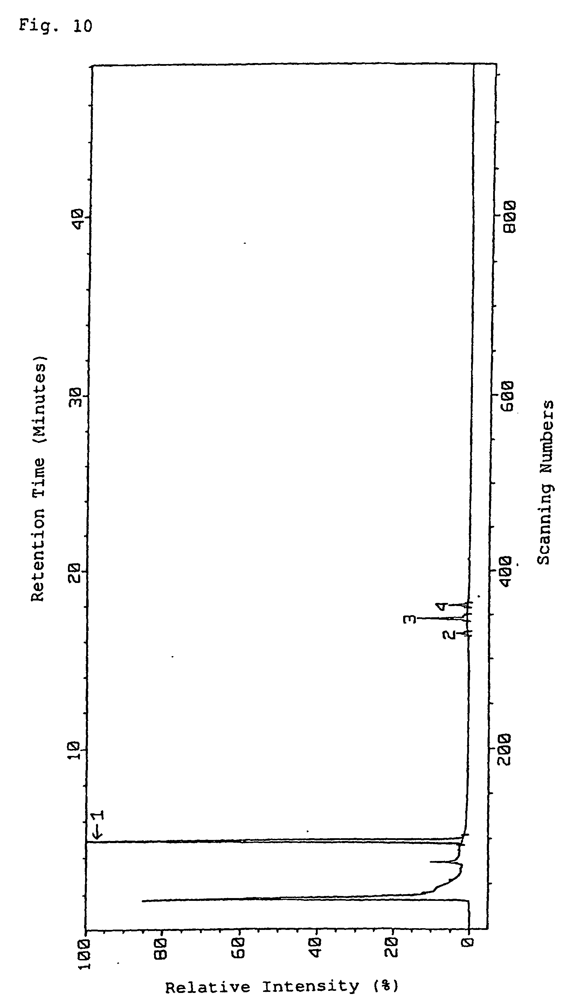

   Figs. 7, 8, 9, 10, 11 and 12 show the total ion chromatograms of the lipid components or sugar components of each of the fraction Nos. 20, 21 and 23. Thus, Figs. 7, 8 and 9 are the drawings which show the total ion chromatograms of the lipid components of each of the fraction Nos. 20, 21 and 23 while Figs. 10, 11 and 12 are the drawings which show the total ion chromatograms of the sugar components of each of the fraction Nos. 20, 21 and 23. In each of the drawings, the ordinate is relative intensity (%) while the upper part and the lower part of the abscissa are retention time (minutes) and scanning numbers, respectively.
   It is shown in Figs. 7 and 10 that peaks of methyl esters of each of tetradecanoic acid (myristic acid) (peak 1 in Fig. 7), hexadecanoic acid (palmitic acid) (peak 3 in Fig. 7), hexadecenoic acid (peak 2 in Fig. 7) and octadecenoic acid (oleic acid) (peak 4 in Fig. 7), a peak derived from glycerol (peak 1 in Fig. 10) and peaks derived from galactose (peaks 2, 3 and 4 in Fig. 10) were detected from the fraction of the fraction No. 20. Further it is shown in Figs. 8 and 11 that peaks of methyl esters of each of tetradecanoic acid (myristic acid) (peak 1 in Fig. 8), hexadecanoic acid (palmitic acid) (peak 3 in Fig. 8), hexadecenoic acid (peak 2 in Fig. 8) and octadecenoic acid (oleic acid) (peak 4 in Fig. 8), a peak derived from glycerol (peak 1 in Fig. 11) and peaks derived from galactose (peaks 2, 3 and 4 in Fig. 11) were detected from the fraction of the fraction No. 21.
   Furthermore it is shown in Figs. 9 and 12 that peaks of methyl esters of each of hexadecanoic acid (palmitic acid) (peak 1 in Fig. 9) and octadecenoic acid (oleic acid) (peak 2 in Fig. 9), a peak derived from glycerol (peak 1 in Fig. 12) and peaks derived from galactose (peaks 2, 3 and 4 in Fig. 12) were detected from the fraction of the fraction No. 23.
   Thus, it was confirmed that the above fractions contained glycerolipid and/or glyceroglycolipid and it was clarified that the glycerolipid and/or glyceroglycolipid have/has an apoptosis inducing activity and an activity of suppressing the growth of cancer cells.
(2) Fraction Nos. 16-26 of the fractions eluted from the Sepharose LH-60 column as mentioned in Example 2-(1) were collected, concentrated and evaporated to dryness, the dried residue was dissolved in chloroform, the solution was treated with a column of 200 ml of Iatrobeads 6RS-8060 (manufactured by Iatron) previously equilibrated with chloroform and the column was eluted with chloroform and then eluted with a mixture of chloroform and acetone in a ratio of 80:20, 60:40, 40:60 and 20:80 successively. As a result, it was confirmed that the fractions eluted with chloroform and with a 80:20 mixture of chloroform and acetone showed a strong apoptosis inducing activity.
   The fractions in which the apoptosis inducing activity was confirmed were subjected to analysis of the components by a method mentioned in Example 1.

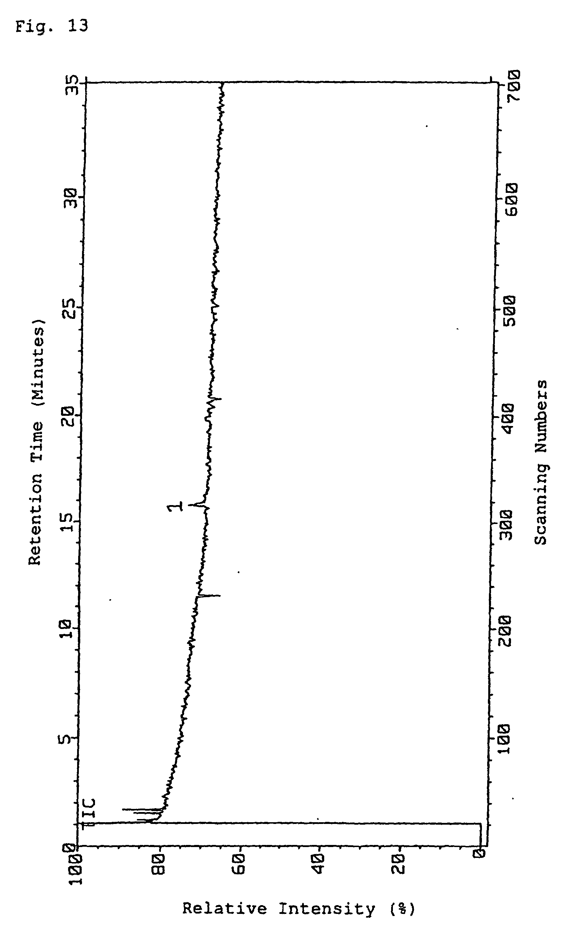

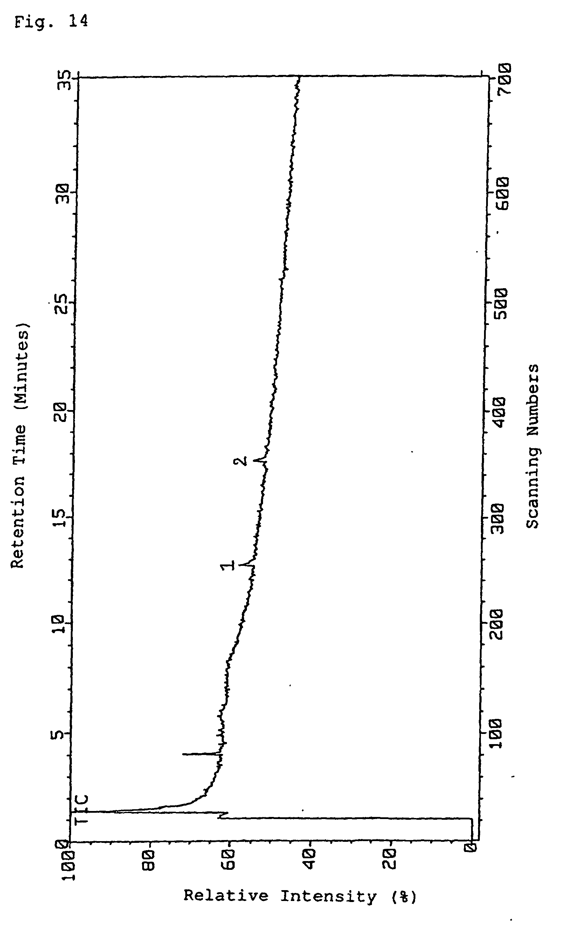

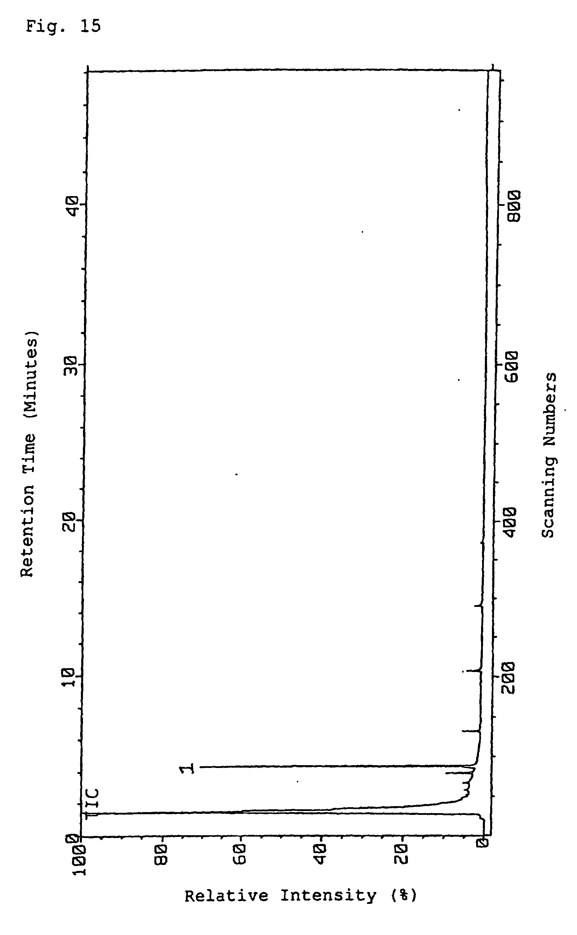

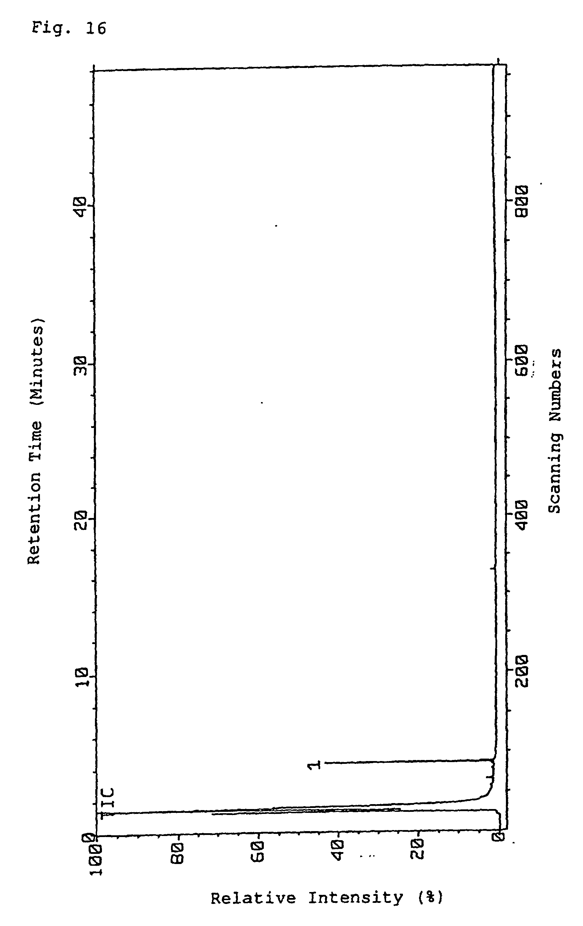

   Figs. 13, 14, 15 and 16 show the total ion chromatograms of the lipid and sugar components in each of the fractions eluted with chloroform and with a 80:20 mixture of chloroform and acetone. Thus, Figs. 13 and 14 are the drawings which show the total ion chromatograms of the lipid components of each of the fractions eluted with chloroform and with a 80:20 mixture of chloroform and acetone while Figs. 15 and 16 are the drawings which show the total ion chromatograms of the sugar components of each of the fractions eluted with chloroform and with a 80:20 mixture of chloroform and acetone. In each of the drawings, the ordinate is a relative intensity (%) while the upper and the lower part of the abscissa are retention time (minutes) and scanning number, respectively.
   It is shown in Figs. 13 and 15 that a peak of methyl ester of hexadecanoic acid (palmitic acid) (peak 1 in Fig. 13) and a peak derived from glycerol (peak 1 in Fig. 15) were detected from the fraction eluted with chloroform while, in Figs. 14 and 16, it is shown that peaks of methyl esters of each of tetradecanoic acid (myristic acid) (peak 1 in Fig. 14) and hexadecanoic acid (palmitic acid) (peaks 2 in Fig. 14) and a peak derived from glycerol (peak 1 in Fig. 16) were detected from the fraction eluted with a 80:20 mixture of chloroform and acetone.
   As such, it was confirmed that the above fractions contained glycerolipid and it was clarified that the glycerolipid had an apoptosis inducing activity and an activity of suppressing the growth of cancer cells.

### Example 3

(1) Water was added to 230 ml of a 40-fold concentrate of the extract derived from *gagome kombu* mentioned in Example 1-(1) to make 910 ml, then 490 ml of ethanol was added thereto and the mixture was stirred for two hours and centrifuged to prepare a substance soluble in 35% ethanol. The 35% ethanolic soluble substance was concentrated and evaporated to dryness, 100 ml of water was added, then 400 ml of a 2:1 mixture of chloroform and methanol were added, the mixture was well stirred and the supernatant liquid separated out thereby was recovered. The lower organic solvent phase was evaporated, water was added thereto to make 100 ml and those operations were repeated for three times. The resulting upper layer solution was concentrated and evaporated to dryness and 10 ml of chloroform was added thereto to give a substance which was soluble in chloroform. This chloroform-soluble substance was concentrated and treated with a column of 200 ml of Iatrobeads 6RS-8060 (manufactured by Iatron) previously equilibrated with chloroform and the column was eluted with chloroform and then with mixtures of chloroform and acetone in ratios of 90:10, 70:30, 40:60 and 20:80 successively.
   As a result, strong apoptosis inducing activity was confirmed in the fractions eluted with chloroform and with a 40:60 mixture of chloroform and acetone.
(2) The fraction eluted with chloroform was spotted on a silica gel plate 60F₂₅₄ (manufactured by Merck) and subjected to a thin layer chromatography using a 9:1 mixture of hexane and ether as a developer whereupon a single spot having an apoptosis inducing activity and an activity of suppressing the growth of cancer cells was detected at the location where the Rf value was about 0.25.
   Analysis of various components was conducted for this spot by a method mentioned in Example 1.

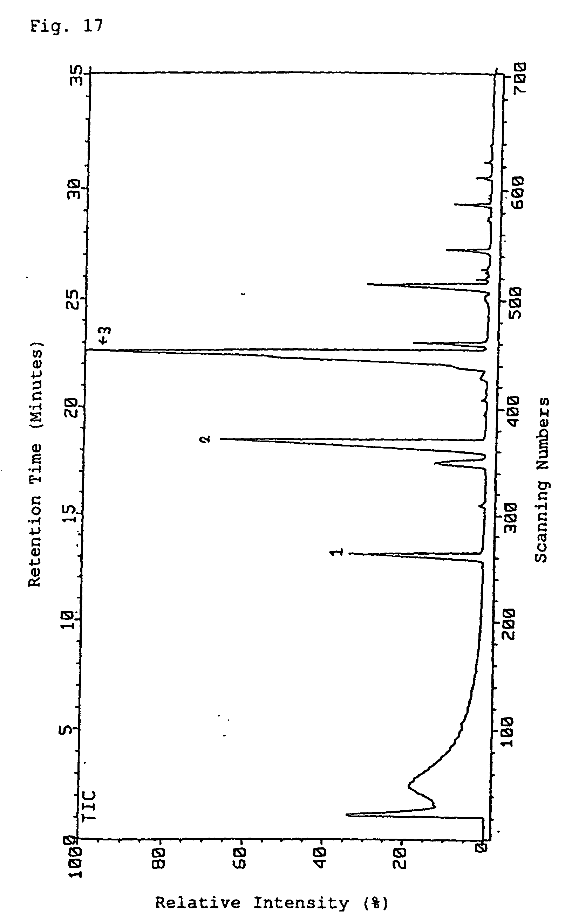

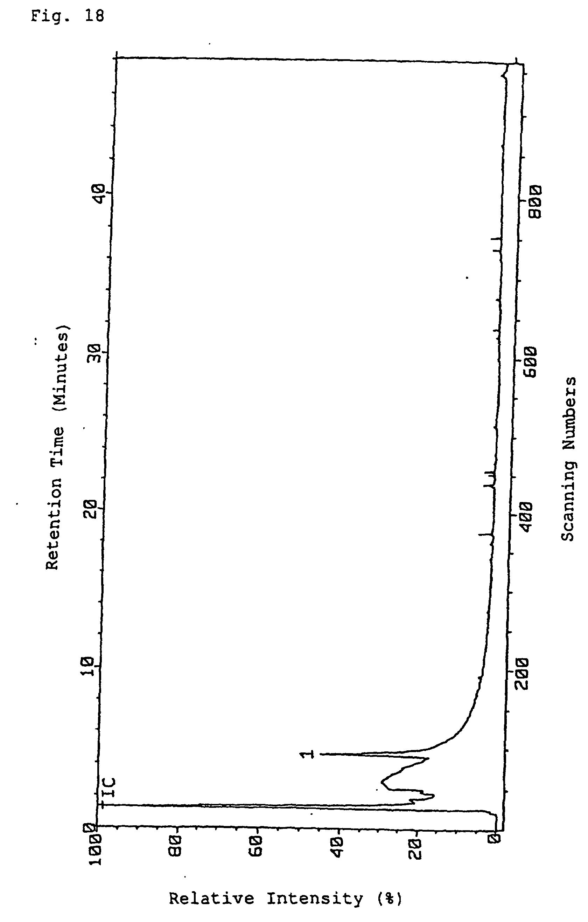

   Figs. 17 and 18 show the total ion chromatograms of the lipid components or sugar components of the spot where the Rf value was about 0.25. Thus, Fig. 17 is a drawing which shows the total ion chromatogram of the lipid components of the spot where the Rf value was about 0.25 while Fig. 18 is a drawing which shows the total ion chromatogram of the sugar components of the spot where the Rf value was about 0.25. In each of the drawings, the ordinate is a relative intensity while the upper and the lower parts of the abscissa are retention time (minutes) and scanning numbers, respectively.
   It is shown from Figs. 17 and 18 that peaks of methyl esters of each of tetradecanoic acid (myristic acid) (peak 1 in Fig. 17), hexadecanoic acid (palmitic acid) (peak 2 in Fig. 17) and octadecenoic acid (oleic acid) (peak 3 in Fig. 17) and a peak derived from glycerol (peak 1 in Fig. 18) were detected from the spot where the Rf value was about 0.25.
(3) The fraction eluted with a 40:60 mixture of chloroform and acetone was subjected to a thin layer chromatography using a 95:12 mixture of chloroform and methanol as a developer whereupon a spot having an apoptosis inducing activity was detected at the location where the Rf value was about 0.33.
   Analysis of various components was conducted for this spot by a method mentioned in Example 1.

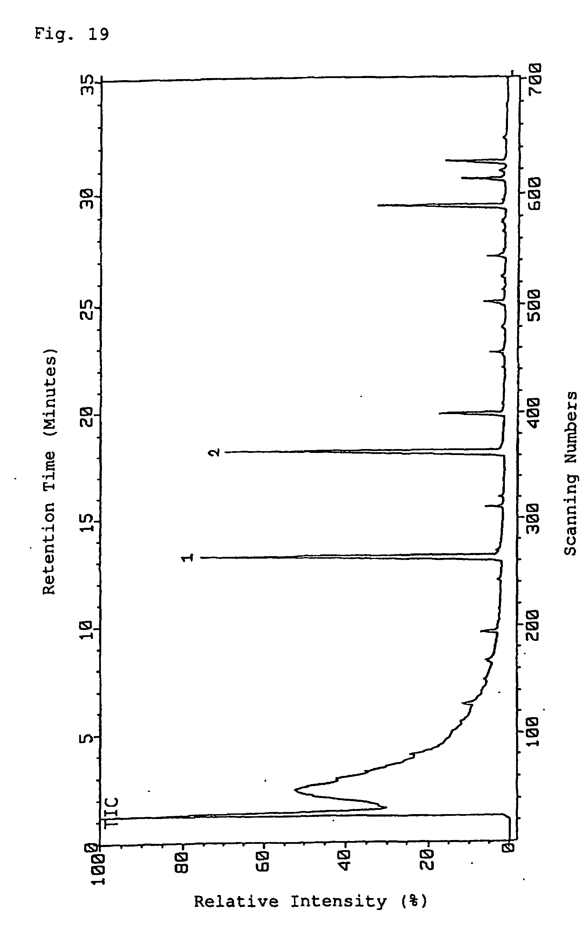

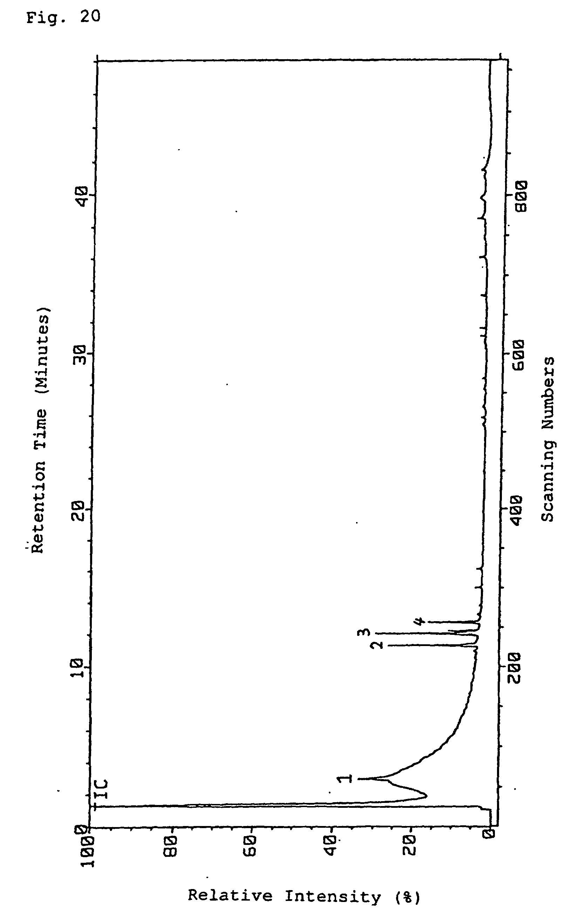

   Figs. 19 and 20 show the total ion chromatogram of the lipid components or sugar components of the spot where the Rf value was about 0.33. Thus, Fig. 19 is a drawing which shows the total ion chromatogram of the lipid components of the spot where the Rf value was about 0.33 while Fig. 20 is a drawing which shows the total ion chromatogram of the sugar components of the spot where the Rf value was about 0.33. In each of the drawings, the ordinate is a relative intensity while the upper and the lower parts of the abscissa are retention time (minutes) and scanning numbers, respectively.
   It is shown from Figs. 19 and 20 that peaks of methyl esters of each of tetradecanoic acid (myristic acid) (peak 1 in Fig. 19) and hexadecanoic acid (palmitic acid) (peak 2 in Fig. 19) and peaks derived from galactose (peaks 2, 3 and 4 in Fig. 20) and a peak derived from glycerol (peak 1 in Fig. 20) were detected from the spot where the Rf value was about 0.33.
   As such, it was confirmed that the spots mentioned in the above-given Example 3-(2) and Example 3-(3) contained the glycerolipid and glyceroglycolipid and it was clarified that the glycerolipid and glyceroglycolipid had an apoptosis inducing activity and an activity of suppressing the growth of cancer cells.

### Example 4

Ten-fold volume of water was added to 2.5 kg of dried gagome kombu crushed into 1 mm cubes and, after said sea tangle pieces were swollen, the supernatant liquid was removed by centrifugation. Ethanol and water were added to the swollen sea tangle so that, finally, 10-fold volume of 60% ethanol were present and then the mixture was stirred at 37°C for three hours and centrifuged to prepare the supernatant liquid. The supernatant liquid was concentrated, then to 750 ml of its aqueous solution were added chloroform and methanol whereby the ratio of chloroform : methanol : water was made 2:4:0.8, the mixture was well stirred and the organic phase solution which was the lower layer was separated.

The lower layer solution was concentrated and evaporated to dryness, a chloroform-soluble substance was prepared therefrom and treated with 200 ml of a column of Iatrobeads 6RS-8060 (manufactured by Iatron) previously equilibrated with chloroform and the column was eluted with chloroform and then with a mixture of chloroform and acetone (40:60), acetone and methanol successively.

Each of the eluted fractions was concentrated, spotted on a silica gel plate 60F₂₅₄ and detection of the glycolipid was conducted using each of a primulin reagent and an orcinol sulfate reagent whereupon the presence of glycolipid(s) in both fractions was confirmed. The result was that the fraction eluted with a solvent having a lower polarity contained higher amounts of lipids and showed stronger activity in terms of an apoptosis inducing action and an action of suppressing the growth of cancer cells.

### Example 5

Dry *gagome kombu* was ground by a mixer. To 0.5 g of the resulting sea tangle powder were added 25 ml of ① 60 mM HCl, ② 1M acetic acid, ③ 1% NaHCO₃, ④ 1% Na₂CO₃, ⑤ 1% Na₂CO₃, ⑥ a 2:1 mixture of chloroform and methanol, ⑦ 75% aqueous solution of ethanol, ⑧ 0.1% SDS or ⑨ 0.01% SDS and extraction was carried out for three hours at 60°C for ①, ②, ③, ④ and ⑦ or at 37°C for all others. Hereinafter, the solutions of ①∼⑨ will be referred to as primary extracts. ① and ② were neutralized with Na₂CO₃, ④ and ⑤ were neutralized with HCl and all others were centrifuged as they were and then subjected to the following operations.
A. To the precipitate were added 25 ml of a 75% aqueous solution of ethanol, extraction was carried out at 60°C for two hours, the supernatant liquid after centrifugation was concentrated and evaporated to dryness and the residue was dissolved in 1 ml of a 75% aqueous solution of ethanol.
B. To 10 ml of the supernatant liquid were added 30 ml of ethanol followed by mixing, the supernatant liquid after centrifugation was concentrated and evaporated to dryness *in vacuo* and the residue was dissolved in 0.4 ml of a 75% aqueous solution of ethanol.
C. The supernatant liquid was evaporated to dryness *in vacuo* and the residue was dissolved in 1 ml of a 75% aqueous solution of ethanol.

The extract prepared as such was diluted with a 75% aqueous solution of ethanol, each 5 µl thereof was added to each of the wells of a 96-well microtiter plate and air-dried, 100 µl of an RPMI 1640 medium containing 10% of fetal calf serum and 5,000 HL-60 cells were added thereto and an activity of suppressing the growth of cancer cells was measured by an MTT method described in Example 7 which will be mentioned later.

The result as shown in Table 1 was obtained. Thus, Table 1 shows the relation between the extracting method and the activity of suppressing the growth of cancer cells where the figures in the table show the diluting rates of the diluted solution having the activity of suppressing the growth of cancer cells. Before extracting with a 75% aqueous solution of ethanol, the powdery sea tangle was treated with the acidic (①A) or alkaline (③A, ④A, ⑤A) solution for primary extract whereby a substance having a two-fold stronger activity of suppressing the growth of cancer cells was extracted as compared with the non-treated case (⑦ C). In addition, an apoptosis inducing activity of those fractions was measured by a method described in Example 8 which will be mentioned later to confirm the apoptosis inducing action of the fractions having an activity of suppressing the growth of cancer cells.

**Table 1**

| Primary Extract | A | B | C |
|---|---|---|---|
| ① | 8 | <2 | |
| ② | 4 | 4 | |
| ③ | 8 | - | |
| ④ | 8 | 4 | |
| ⑤ | 8 | 2 | |
| ⑥ | | | 8 |
| ⑦ | | | 4 |
| ⑧ | | 2 | |
| ⑨ | | <2 | |

### Example 6

(1) *Lyophyllum ulmarium* M-8171 (FERM BP-1415) was cultivated according to a method mentioned in the Japanese Examined Patent Publication (Kokoku) Hei-06/34,660 to prepare its fruit body. Incidentally, the resulting fruit body is as same that the fruit body which is commercially available as "Yamabiko Honshimeji" in the Japanese market. In addition, *Lyophyllum decastes* K-3304 (FERM BP-4348) was cultivated according to a method mentioned in the Japanese Laid-Open Patent Publication (Kokai) Hei-03/65,261 to prepare its fruit body (Japanese name, *hatakeshimeji*). Each of those fruit bodies and commercially available *maitake, enokitake, shiitake* and *nameko* was freeze dried and then the resulting freeze-dried products were ground to give the ground products thereof.
   A 75% aqueous ethanol (25 ml) was added to each 0.5 g of the above-prepared ground products and extraction was carried out at room temperature for two hours. After an extract was obtained by means of centrifugation, said extract was concentrated and evaporate to dryness *in vacuo* to give a dry product. This dry product was dissolved in 1 ml of water to prepare an ethanolic extract.
(2) An activity of suppressing the growth of cancer cells of the ethanolic extract mentioned in the above Example 6-(1) was measured.
   Each of the ethanolic extracts was sterilized by filtering through a GD/X PES filter (manufactured by Whatman) and dilution series were prepared using sterilized water. Each 10 µl of the diluted solution were placed in each of the wells of a 96-well microtiter plate, 100 µl of an RMPI 1640 medium containing 10% of fetal calf serum and containing 5,000 HL-60 cells were added thereto and incubation was conducted at 37°C for 48 hours in the presence of 5% carbon dioxide gas. The shape of the cells was observed under an optical microscope, 10 µl of a phosphate-buffered aqueous saline solution containing 5 mg/ml of 3-(4,5-dimethylthiazol-2-yl)-2,5-diphenyltetrazolium bromide (MTT; manufactured by Sigma) was added, incubation was continued for four hours more and the state of growth of the cells was observed under a microscope. In the meanwhile, 100 µl of 2-propyl alcohol containing 0. 04N HCl were added followed by well stirring and the absorbance at 590 nm was measured and adopted as a degree of growth of cells.
   With regard to the activity of those ethanolic extracts of mushrooms for suppressing the growth of cancer cells, the cells were found to be killed by a 30-fold diluted solution of *Lyophyllum ulmarium* and a 10-fold diluted solution of *Lyophyllum decastes* whereby a strong activity of suppressing the growth of cancer cells was noted and apoptic bodies were found. In the case of 3-fold diluted solution of *maitake, enokitake, shiitake* and *nameko*, cells were killed while, in the case of 10-fold diluted solutions, apoptic bodies were found whereby each of the ethanolic extracts of those mushrooms showed strong activity of suppressing the growth of cancer cells and apoptosis inducing action.
(3) With regard to the ethanolic extract of *Lyophyllum ulmarium*, analysis of the components was carried out by a method mentioned in Example 1.

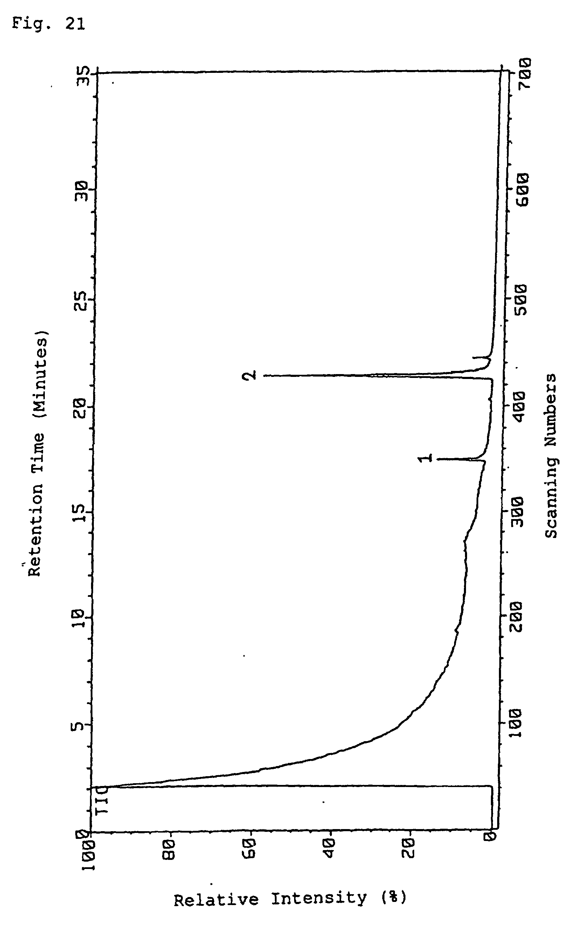

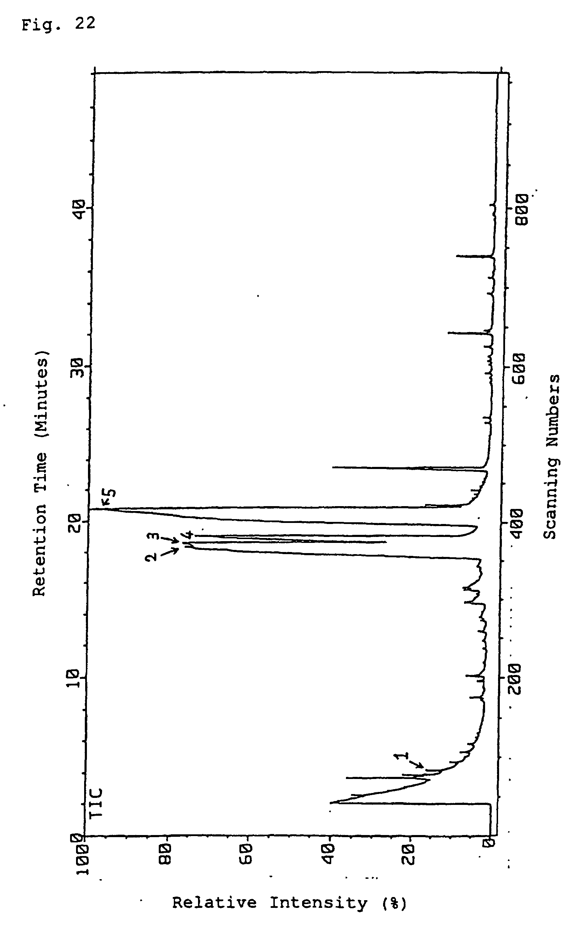

   Figs. 21 and 22 show the total ion chromatograms of lipid components or sugar components in the ethanolic extract of *Lyophyllum ulmarium*. Thus, Fig. 21 is a drawing which shows the total ion chromatogram of the lipid components in the ethanolic extract of *Lyophyllum ulmarium* while Fig. 22 is a drawing which shows the total ion chromatogram of the sugar components in the ethanolic extract of *Lyophyllum ulmarium*. In each of the drawings, the ordinate shows a relative intensity (%) while the upper and the lower parts of the abscissa show the retention time (minutes) and scanning numbers, respectively.
   Figs. 21 and 22 show that peaks of methyl esters of each of hexadecanoic acid (palmitic acid) (peak 1 in Fig. 21) and cis-9,cis-12-octadecadienoic acid (linoleic acid) (peak 2 in Fig. 21), peaks derived from glucose (peaks 2, 3 and 4 in Fig. 22), a peak derived from mannitol (peak 5 in Fig. 22) and a peak derived from glycerol (peak 1 in Fig. 22) were detected in the ethanolic extract of *Lyophyllum ulmarium*.
   Presence of glycerolipid and/or glyceroglycolipid was confirmed in the ethanolic extracts of other mushrooms as well.

### Example 7

Frozen ground *Lyophyllum ulmarium* (5 g) mentioned in Example 6 was suspended in 250 ml of a 75% aqueous solution of ethanol and the suspension was shaken at room temperature for two hours to separate into a supernatant liquid and a precipitate by centrifugation whereupon an extract is prepared. The extract is concentrated and evaporated to dryness using a rotary evaporator (30°C) and the residue was re-dissolved in 20 ml of a 75% aqueous solution of ethanol to give a concentrated extract of *Lyophyllum ulmarium*. To a part of this concentrated extract of *Lyophyllum ulmarium* were added one-half volume of water and the same volume of chloroform and the resulting suspension was allowed to stand to fractionate into the upper and the lower layers. Each of the fractionated upper and lower layers was concentrated and evaporated to dryness, the residue was re-dissolved in one-half volume (of the used concentrated extract of *Lyophyllum ulmarium*) of a 75% aqueous solution of ethanol and the activity for suppressing the growth of cancer cells of each of the fractions was measured as follows.

Diluted series of the re-dissolved solution of each of the fractions in a 75% aqueous ethanol were prepared using a 75% aqueous solution of ethanol. Each 5 µl of the diluted solutions was placed in a 96-well microtiter plate and dried, 100 µl of an RPMI 1640 medium containing 10% fetal calf serum and containing 5,000 HL-60 cells (ATCC CCL-240) was added thereto and incubation was conducted at 37°C for 48 hours. After the shape of the cells was observed under an optical microscope, 10 µl of buffered aqueous saline solution containing 5 mg/ml of MTT was added, incubation was conducted for four hours more and the state of growth of the cells was observed under a microscope. In the meanwhile, 100 µl of 2-propyl alcohol containing 0.04N HCl were added followed by well stirring and the absorbance at 590 nm was measured and adopted as a degree of growth of cells (an MTT method).

The result was that the cells were killed by a 20-fold diluted solution of the above fraction from the lower layer whereupon a strong activity for suppressing the growth of cancer cells was noted and apoptic bodies were confirmed. Thus, this concentrated extract of *Lyophyllum ulmarium* was subjected to a fractionation by chloroform by the same manner, re-dissolved in a mobile phase for a silica gel column chromatography instead of dissolving in an 75% aqueous solution of ethanol and subjected to a silica gel column chromatography. The silica gel column chromatography was conducted under the two conditions of using a mobile phase consisting of chloroform : methanol : water = 65:25:4 (condition No. 1) and that consisting of chloroform : methanol = 65:25 (condition No. 2) independently. Each of the fractions of the silica gel column chromatography was concentrated and evaporated to dryness, the residue was re-dissolved in a 75% aqueous solution of ethanol in 1/20 of the initial liquid volume, its activity for suppressing the growth of cancer cells was measured by the same MTT method as above and the fraction where the activity was confirmed was analyzed by means of a TLC (chloroform : methanol : water = 65:25:4). The result was that, under the condition No.1, the activity was available in a fraction A in which spots reactive to a primulin reagent (lipids), spots reactive to a ninhydrin reagent (amino group), spots reactive to an orcinol sulfate reagent (sugars) and spots reactive to Dittmer reagent (phospholipids) were present and in a fraction B in which spots reactive to a primulin reagent and spots reactive to an orcinol sulfate reagent were present. Under the condition No. 2, the activity was available in a fraction C in which spots reactive to an orcinol sulfate reagent were present, in a fraction D in which spots reactive to a primulin reagent and spots reactive to an orcinol sulfate reagent were present and in a fraction E in which spots reactive to a primulin reagent, spots reactive to a ninhydrin reagent and spots reactive to an orcinol sulfate reagent were present. Further, the fraction A under the condition No. 1 was collected, concentrated and evaporated to dryness, dissolved in a mobile phase and subjected to a silica gel column chromatography. A mixture of chloroform and methanol in a ratio of 95:5 was used as a mobile phase (condition No. 3). Each of the fractions was assayed by an MTT method by the same manner as before and the fractions where the activity was confirmed were analyzed by a TLC (chloroform : methanol = 95:5). The result was that, under the condition No. 3, the activity was available in a fraction F in which spots reactive to a primulin reagent were present, in a fraction G in which spots reactive to a primulin reagent and spots reactive to an orcinol sulfate reagent were present and in a fraction H in which spots reactive to an orcinol sulfate reagent were present. The apoptosis inducing activity of those fractions which were active for suppressing the growth of cancer cells was confirmed by the formation of apoptic bodies.

### Example 8

The ground freeze-dried product (1 g) of *Lyophyllum ulmarium* mentioned in Example 6 was suspended in 50 ml of a 75% aqueous solution of ethanol or a 50% aqueous solution of ethanol, shaken at 37°C for two hours and centrifuged to separate into a supernatant liquid and a precipitate whereupon each extract was prepared. Each of the extract was concentrated and evaporated to dryness using a rotary evaporator (30°C) and re-dissolved in 8 ml of a 75% aqueous solution of ethanol or a 50% aqueous solution of ethanol to give a concentrated extract of *Lyophyllum ulmarium*.

Each of the concentrated extracts of *Lyophyllum ulmarium* was diluted with a 75% or 50% aqueous solution of ethanol. The diluted solution was assayed by an MTT method mentioned in Example 7. Death of cell was noted in a section where a 10-fold diluted solution of the extract with a 75% or 50% aqueous solution of ethanol and an activity for suppressing the growth of cancer cells was confirmed. Thus, it was confirmed that, under the conditions of extracting with a 75% aqueous solution of ethanol and a 50% aqueous solution of ethanol, substances which are active for suppressing the growth of cancer cells were extracted. Further, an apoptosis inducing activity was measured using those concentrated extracts of *Lyophyllum* *ulmarium*. Thus, 25 µl of each of the above-mentioned concentrated extracts was used as a sample, ethanol was evaporated therefrom, the residue was added to 2.5 × 10⁵ HL-60 cells/4.5 ml which were incubated in an RPMI 1640 medium containing 10% of fetal calf serum and incubation was conducted at 37°C for 48 hours. At the same time, control samples were prepared by adding distilled water and an Actinomycin D solution (10 µg/ml) which was known to have an apoptosis inducing activity. Formation of apoptic bodies, contraction of cells and aggregation of nuclei were observed by naked eye under an optical microscope and the numbers of living cells were counted. When such a phenomenon was observed and a reduction in living cell numbers was noted, the sample was judged to have an apoptosis inducing activity. The result was that the extracts with a 75% aqueous solution of ethanol and a 50% aqueous solution of ethanol contained an apoptosis inducing substance.

### Example 9

(1) A 75% aqueous solution (25 ml) of ethanol was added to 0.5 g of each of freeze-dried powder of commercially available *hiratake* (*Pleurotus ostreatus*) and common mushroom (*Agaricus bisporus* or the like) and extraction was conducted at 37 °C for two hours. The supernatant liquid after centrifugation was concentrated and evaporated to dryness *in vacuo*, the residue was dissolved in 1 ml of a 75% aqueous solution of ethanol and the solution was diluted with a 75% aqueous solution of ethanol. To 1 µl of the diluted solution was added 100 µl of an RPMI 1640 medium containing 10% of fetal calf serum and containing 5,000 HL-60 cells and the mixture was subjected to a measurement for an activity of suppressing the growth of cancer cells by an MTT method mentioned in Example 7 and also to a measurement for an apoptosis inducing activity by a method mentioned in Example 8. The result was that a two-fold diluted solution of *hiratake* and a non-diluted solution of common mushroom showed both activities.
(2) To 40 g of *bunashimeji* (*Lyophyllum ulmarium*) mentioned in Example 6 were added 0 (0%), 50 (25%), 100 (50%) or 150 ml (75%) of ethanol and 160, 110, 60 or 10 ml of water, the mixture was homogenized for 30 seconds in a mixer, shaken at room temperature for two hours and filtered to give an extract. The extract was diluted with a 75% aqueous solution of ethanol and an activity of suppressing the growth of cancer cells and an apoptosis inducing activity were measured by an MTT method mentioned in Example 7 and by a method mentioned in Example 8, respectively. The result was that both activities were available in a two-fold diluted solution of the extracts with 0, 25 and 50% aqueous solutions of ethanol and also in a five-fold diluted solutions of a 75% aqueous solution of ethanol.
To 40 grams of the above-mentioned *bunashimeji* was cut in 3-5 mm cubes were added 50 (25%), 100 (50%) or 150 ml (75%) of ethanol and 110, 60 or 10 ml of water and the mixture was shaken at room temperature for two hours and filtered to give an extract. The extract was diluted with a 75% aqueous solution of ethanol and its activity of suppressing the growth of cancer cells was measured by an MTT method mentioned in Example 7 while its apoptosis inducing activity was measured by a method mentioned in Example 8. The result was that both activities were available in the original (non-diluted) solution of the extract with 25% ethanol and in the two-fold diluted solutions of the extracts with 50% and 75% ethanol.
(3) Commercially available *shiitake* (*Cortinellius shiitake*) was freeze-dried and ground and the resulting powder was extracted for two hours under the conditions as mentioned in Table 2.

**Table 2**

| | *Shiitake* Powder | Concentration of Ethanol | Amount of Liquid for Extraction | Temperature for Extraction |
|---|---|---|---|---|
| ① | 0.5 g | 25% | 20 ml | 60°C |
| ② | 0.5 g | 75% | 10 ml | room temperature |
| ③ | 0.5 g | 50% | 10 ml | room temperature |
| ④ | 0.5 g | 75% | 25 ml | room temperature |

After the extraction, insoluble matters were removed by centrifugation and the supernatant was concentrated and evaporated to dryness *in vacuo* and dissolved in 1 ml of an aqueous solution of ethanol having the same concentration as that used for the extraction. The solution was diluted with a 75% aqueous solution of ethanol and its activity of suppressing the growth of cancer cells was measured by an MTT method mentioned in Example 7 while its apoptosis inducing activity was measured by a method mentioned in Example 8 whereupon both activities were available in a ten-fold diluted solution of ①, a two-fold diluted solution of ②, a five-fold diluted solution of ③ and a five-fold diluted solution of ④.
(4) Commercially available *shiitake* was separated into caps and stems. Each of them was freeze-dried separately and ground. To 0.5 g of the resulting powder was added 25 ml of a 75% aqueous solution of ethanol followed by extracting at 37°C for two hours. The supernatant liquid after centrifugation was concentrated and evaporated to dryness *in vacuo*, the residue was dissolved in 1 ml of a 75% aqueous solution of ethanol and the resulting solution was diluted with a 75% aqueous solution of ethanol. An activity of suppressing the growth of cancer cells and an apoptosis inducing activity of each diluted solution were measured by an MTT method mentioned in Example 7 and by a method mentioned in Example 8, respectively whereupon both activities were found to be available in a two-fold diluted solution of the extract of the caps and in a 20-fold diluted solution of the extract of the stems. Dried Chinese *shiitake* of a high grade and also caps and stems of Chinese *shiitake* of a medium grade were ground by a mixer as well and the same extraction and same measurement for apoptosis inducing activity were conducted whereupon five-fold diluted solutions of all of the extracts of high-grade one and caps and stems of middle-grade one showed both activities.

### Example 10

(1) To 0.5 g of commercially available *matcha* (powdered green tea) was added 25 ml of water of a 75% aqueous solution of ethanol and the mixture was shaken for two hours at room temperature. The extract was centrifuged and the supernatant liquid was concentrated and evaporated to dryness *in vacuo* and the residue was dissolved in 1 ml of water.
   The extract was diluted from 1-fold to 100-fold in a series. Ten µl of each of the diluted solutions and 100 µl of an RPMI 1640 medium containing 10% of fetal calf serum and containing 5,000 HL-60 cells was added to each of the wells of a 96-well microtiter plate and incubated for 48 hours and the state of growth of the cells was observed under an optical microscope. The result was that cells were killed in the cases of all of the diluted solutions in both extracts with water and a 75% aqueous solution of ethanol and that both aqueous and ethanolic extracts of tea showed a strong activity for suppressing the growth of cancer cells. In addition, their apoptosis inducing action was confirmed due to formation of the apoptic bodies.
(2) Analysis of various components in the ethanolic extract of *matcha* mentioned in Example 10-(1) was conducted by a method mentioned in Example 1.

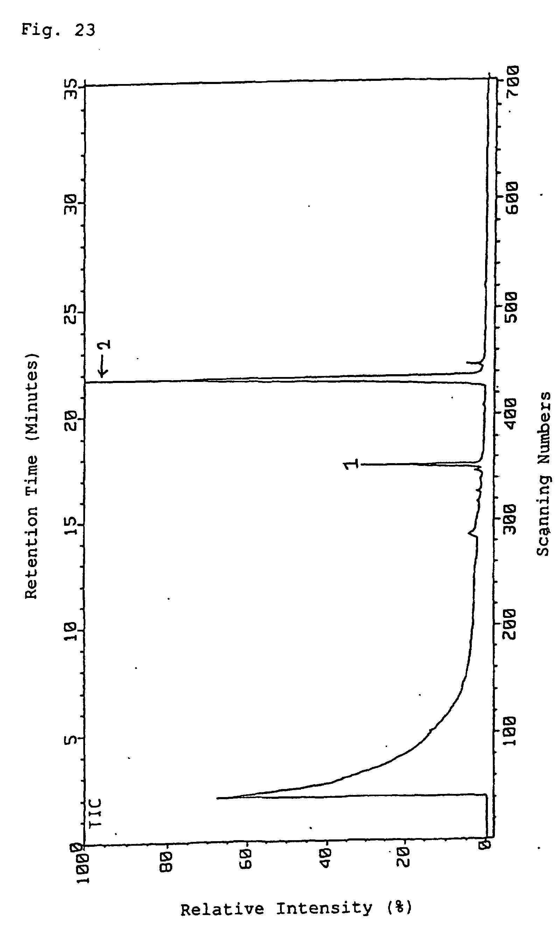

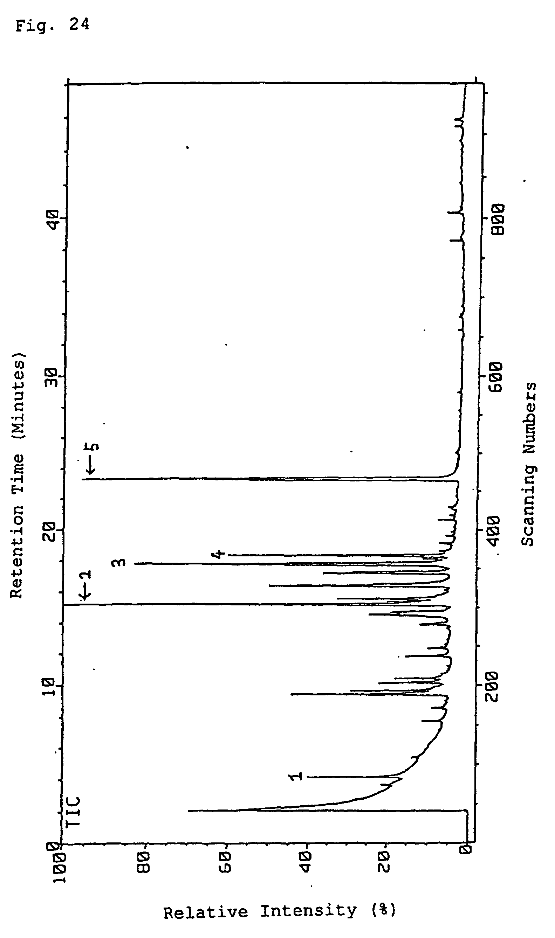

   Figs. 23 and 24 show the total ion chromatograms of the lipid components or the sugar components of the ethanolic extract of *matcha*. Thus, Fig. 23 is a drawing which shows the total ion chromatogram of the lipid components of the ethanolic extract of *matcha* while Fig. 24 is a drawing which shows the total ion chromatogram of the sugar components of the ethanolic extract of *matcha*. In each of the drawings, the ordinate shows a relative intensity (%) while the upper and the lower parts of the abscissa show retention time (minutes) and scanning numbers, respectively.
   It is shown in Figs. 23 and 24 that there were peaks of methyl esters of hexadecanoic acid (palmitic acid) (peak 1 in Fig. 23) and 9,12,15-octadecatrienoic acid (linolenic acid) (peak 2 in Fig. 23), peaks derived from glucose (peaks 3 and 4 in Fig. 24) and a peak derived from glycerol (peak 1 in Fig. 24) were detected in the ethanolic extract of *matcha* whereby the presence of glycerolipid and/or glyceroglycolipid were/was confirmed. A peak derived from myoinositol (peak 5 in Fig. 24) and a peak derived from quinic acid (peak 2 in Fig. 24) were detected as well.

### Example 11

(1) To 0.5 g of rice bran was added water, a 75% aqueous solution of ethanol or a 90% aqueous solution of ethanol followed by shaking at room temperature for two hours. The extract was centrifuged, the supernatant liquid was concentrated and evaporated to dryness in vacuo and the residue was dissolved in 1 ml of water. In the case of an extract with a 90% aqueous solution of ethanol, the water insoluble matters were dissolved in ethanol. Those samples were diluted and their activity for suppressing the growth of cancer cells and their apoptosis inducing activity were measured by an MTT method mentioned in Example 7 and by a method mentioned in Example 8, respectively. The result was that both activities were available in five-fold diluted solution of the aqueous extract, in non-diluted solution of the extract with 75% ethanol and in ten-fold diluted solution of ethanol-soluble fraction of the extract with 90% ethanol whereupon the ethanolic extracts of rice bran showed a strong action of suppressing the growth of cancer cells and apoptosis inducing action.
(2) Analysis of various components in the extract with 75% ethanol of rice bran mentioned in Example 11-(1) was conducted by a method mentioned in Example 1.

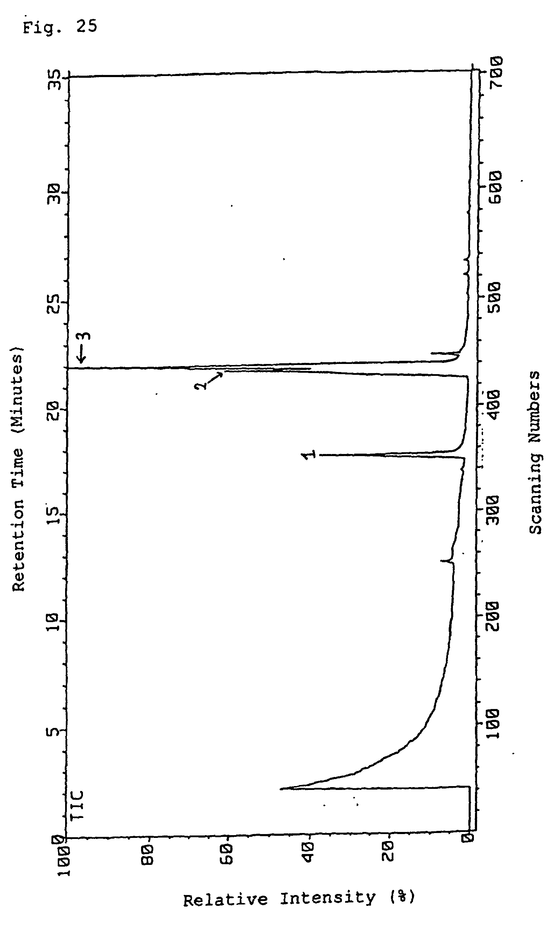

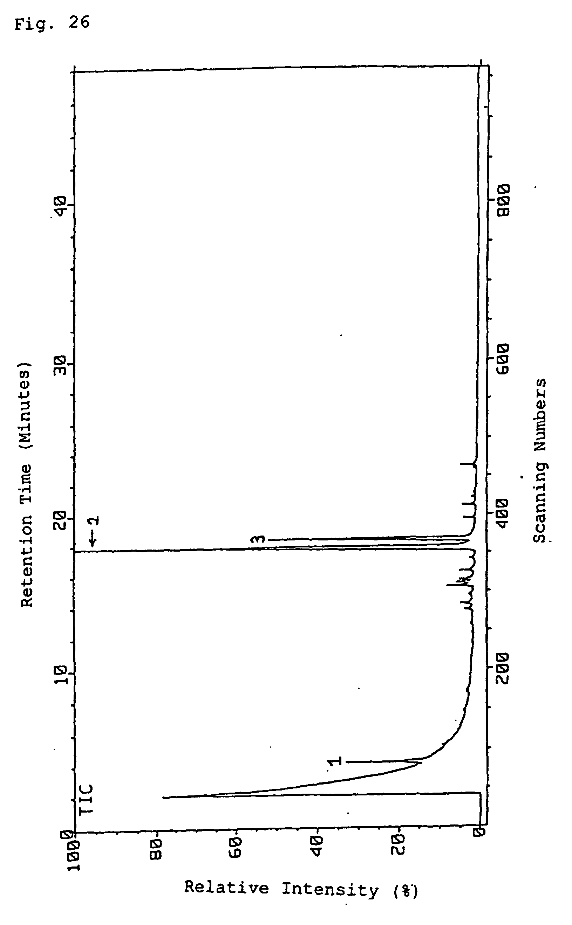

   Figs. 25 and 26 show the total ion chromatograms of the lipid components or sugar components of the 75% ethanolic extract of rice bran. Thus, Fig. 25 is a drawing which shows the total ion chromatogram of the lipid components of the 75% ethanolic extract of rice bran while Fig. 26 is a drawing which shows the total ion chromatogram of the sugar components of the 75% ethanolic extract of rice bran. In each of the drawings, the ordinate shows a relative intensity (%) while the upper and the lower parts of the abscissa show retention time (minutes) and scanning numbers, respectively.
   It is shown in Figs. 25 and 26 that peaks of methyl esters of hexadecanoic acid (palmitic acid) (peak 1 in Fig. 25), octadecenoic acid (oleic acid) (peak 3 in Fig. 25) and cis-9,cis-12-octadecadienoic acid (linoleic acid) (peak 2 in Fig. 25), peaks derived from glucose (peaks 2 and 3 in Fig. 26) and a peak derived from glycerol (peak 1 in Fig. 26) were detected in the 75% ethanolic extract of rice bran whereby the presence of glycerolipid and/or glyceroglycolipid was confirmed.

### Example 13

Monogalactosyl diglyceride (manufactured by Wako Pure Chemical) derived from spinach, monogalactosyl diglyceride (manufactured by Funakoshi) derived from wheat and digalactosyl diglyceride derived from wheat were suspended in a few amounts of n-decane, the suspension was further dispersed by means of an ultrasonic treatment and ethanol was added thereto to make the ratio of n-decane ethanol = 2:98. The solution was diluted to an extent of 50-fold with an RPMI 1640 medium, 0.5 ml thereof was added to 4.5 ml of a medium containing 2.5 × 10⁵ HL-60 cells which was incubated in an RPMI 1640 containing 10% of fetal calf serum. This was incubated at 37°C for 48 hours and an apoptosis inducing action of each of them was measured by a method mentioned in Example 1-(4) whereupon a strong apoptosis inducing action and an activity of suppressing the growth of cancer cells were confirmed.

### Example 14

Commercially available cabbage, peels of eggplant, peeled-off eggplant and spinach leaves were freeze-dried, ground, extracted by the same manner as in Example 9-(4) and measurement of an activity for suppressing the growth of cancer cells and an apoptosis inducing activity were measured by an MTT method mentioned in Example 7 and a method mentioned in Example 8, respectively whereupon both activities were found to be available in two-fold diluted solutions of extracts of cabbage, eggplant peels and spinach leaves and in a five-fold diluted solution of peeled-off eggplant.

### Merit of the Invention

In accordance with the present invention, an apoptosis inducing agent in which glycerolipid (such as the glycerolipid having a strong apoptosis inducing action derived from plants, microorganisms or animals) are/is effective component(s) can be offered and, in addition, an anticancer agent due to said apoptosis inducing action can be offered as well. Said glycerolipid are/is abundantly present in membrane components of plants, microorganisms or animals and can be efficiently extracted by an aqueous ethanol or the like whereupon the effective component (s) of the present invention can be obtained easily. Before extracting with a solvent, an acidic or alkaline treatment may be conducted so that an extracting efficiency can be improved. Further, polarity of the extracting solvent, chromatographic carrier for separation, etc. can be selected depending upon the hydrophobicity thereof whereby desired glycerolipid can be selectively manufactured. Food or beverage where the glycerolipid which are/is apoptosis inducing compound (s) of the present invention extracted and/or purified particularly from edible plants, microorganisms or animals are/is contained therein, added thereto and/or diluted therein is very useful as a healthy food because its daily intake improves the health.

## Claims

1. The use of glycerolipid consisting of fatty acid and glycerol residues in the manufacture of a medicament for the treatment of cancer.

2. The use of glycerolipid consisting of fatty acid and glycerol residues in the manufacture of a food or beverage for the treatment of cancer.

3. The use as set forth in claim 1 or 2 wherein the said glycerolipid is derived from plants, microorganisms or animals.

4. The use as set forth in claim 3 wherein said glycerolipid is derived from tea, mushroom or cereal residue.

## Patentansprüche

1. Verwendung von Glycerolipid bestehend aus Fettsäure und Glycerolresten bei der Herstellung eines Medikaments für die Behandlung von Krebs.

2. Verwendung von Glycerolipid bestehend aus Fettsäure und Glycerolresten bei der Herstellung eines Lebensmittels oder Getränks für die Behandlung von Krebs.

3. Verwendung nach Anspruch 1 oder 2, wobei das genannte Glycerolipid von Pflanzen, Mikroorganismen oder Tieren abgeleitet ist.

4. Verwendung nach Anspruch 3, wobei das genannte Glycerolipid von Tee-, Pilz- oder Getreideresten abgeleitet ist.

## Revendications

1. Utilisation d'un glycérolipide consistant en résidus d'acide gras et de glycérol dans la fabrication d'un médicament pour le traitement du cancer.

2. Utilisation d'un glycérolipide consistant en résidus d'acide gras et de glycérol dans la fabrication d'un aliment ou d'une boisson pour le traitement du cancer.

3. Utilisation telle que stipulée dans la revendication 1 ou 2, dans laquelle ledit glycérolipide est dérivé de plantes, de micro-organismes et d'animaux.

4. Utilisation telle que stipulée dans la revendication 3, dans laquelle ledit glycérolipide est dérivé de thé, de champignon ou d'un résidu de céréale.
